(19) 

**Europäisches Patentamt**
**European Patent Office**
**Office européen des brevets**

(11)  **EP 4 613 879 A1**

(12) **EUROPEAN PATENT APPLICATION**
published in accordance with Art. 153(4) EPC

(43) Date of publication:
**10.09.2025 Bulletin 2025/37**

(21) Application number: **23886232.0**

(22) Date of filing: **31.10.2023**

(51) International Patent Classification (IPC):
***C12Q 1/6886*** (2018.01)

(52) Cooperative Patent Classification (CPC):
**C12Q 1/6886**

(86) International application number:
**PCT/KR2023/017173**

(87) International publication number:
**WO 2024/096536 (10.05.2024 Gazette 2024/19)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **31.10.2022 KR 20220142051**

(71) Applicant: **GC Genome Corporation
Yongin-si, Gyeonggi-do 16924 (KR)**

(72) Inventors:
• **CHO, Eun-Hae
  Yongin-si Gyeonggi-do 16924 (KR)**
• **KIM, Minjung
  Yongin-si Gyeonggi-do 16924 (KR)**
• **PARK, Jun-Tae
  Yongin-si Gyeonggi-do 16924 (KR)**

(74) Representative: **Mewburn Ellis LLP
Aurora Building
Counterslip
Bristol BS1 6BX (GB)**

(54) **DNA METHYLATION MARKER FOR DIAGNOSING LUNG CANCER AND USES THEREOF**

(57)    The present invention relates to DNA methylation markers for diagnosing lung cancer and the use thereof, and more specifically, to a combination of DNA methylation markers capable of determining the presence or absence of lung cancer and the use thereof. The DNA methylation markers for diagnosing lung cancer according to the present invention are capable of diagnosing lung cancer with high accuracy using only DNA methylation information of a blood sample without using a lung cancer tissue sample, and thus may be useful for early diagnosis of lung cancer.

FIG. 1

EP 4 613 879 A1

## Description

## Technical Field

The present invention relates to DNA methylation markers for diagnosing lung cancer and the use thereof, and more specifically, to a combination of DNA methylation markers capable of determining the presence or absence of lung cancer and the use thereof.

## Background Art

Lung cancer is the most common cause of cancer-related death worldwide. Despite significant advances in the detection and treatment of lung cancer over the past two decades, the prognosis for patients with the disease still remains poor because the combined 5-year survival rate for all stages of lung cancer is 10% to 15%. The poor prognosis for lung cancer patients is a result of some recurrence, which occurs in approximately 20 to 50% of patients who had undergone curative surgical resection with adequate lymph node dissection. Even for patients with stage 1 lung cancer, the 5-year survival rate is less than 50% because the cancer has spread to nearby lymph nodes or other organs before it is detected.

Lung cancer is classified into small cell lung cancer (hereinafter also referred to as SCLC) and non-small cell lung cancer (hereinafter also referred to as NSCLC). Non-small cell lung cancer is further classified into histological types such as squamous cell carcinoma, adenocarcinoma, large cell carcinoma, and adenosquamous cell carcinoma. Small cell carcinoma has a poorer prognosis after surgery than non-small cell carcinoma, and anticancer drugs are usually used as major therapeutic agents. For non-small cell carcinoma, surgery is generally used in stages 1 and 2, and anticancer drugs are used as major therapeutic agents in advanced cases. Non-small cell lung cancer, which accounts for about 80% of the above-described lung cancer, is a cancer that is difficult to cure because the stage and treatment method are determined based on the size of the tumor mass, whether it has invaded surrounding tissues, the degree of lymph node invasion, and whether it has metastasized to distant organs, and because treatment methods other than surgery have little effect. The reason for such a poor prognosis and high mortality rate is the lack of effective agents or methods capable of effectively diagnosing lung cancer. Therefore, early diagnosis and treatment are very important in lung cancer, which is an incurable disease.

In order to accurately diagnose cancer, it is important not only to identify a mutated gene, but also to understand the mechanism by which the mutation occurs. Previously, studies were conducted focusing on mutations in the coding sequence of the gene, that is, micro-changes such as point mutations, deletions, and insertions, or on macroscopic chromosomal abnormalities. However, in recent years, extragenic changes have been reported to be as important as these mutations, and a representative example thereof is the methylation of promoter CpG islands.

In the genomic DNA of mammal cells, a fifth base is present, in addition to A, C, G and T, and is 5-methylcytosine (5-mC), in which a methyl group is attached to the fifth carbon of a cytosine ring. 5-mC is always attached only to the C of a CG dinucleotide (5'-mCG-3'), and this CG is often denoted as CpG. The C of CpG is mostly methylated by attachment of a methyl group. This methylation of CpG inhibits the expression of repetitive sequences in the genome, such as Alu or transposons, and CpG is the site where extragenic changes most frequently occur in mammalian cells. 5-mC of this CpG is naturally converted into T through deamination. Accordingly, CpG appears in the mammalian genome with a frequency of only 1%, which is much lower than the normal frequency thereof ($\frac{1}{4} \times \frac{1}{4}$=6.25%).

There are regions called CpG islands in which CpGs are exceptionally dense. A CpG island is 0.2 to 3 kb in length, and is a highly concentrated region in which the distribution percentage of C and G bases is greater than 50% and the distribution percentage of CpG is 3.75% or more. About 45,000 CpG islands appear in the entire human genome, and are intensively found in the promoter region, which regulates gene expression. Indeed, CpG islands appear in promoters of housekeeping genes, which account for about half of human genes (Cross S. et al., Curr. Opin. Gene Develop., 5:309, 1995). Accordingly, attempts have recently been actively made to investigate promoter methylation of tumor-related genes in blood, sputum, saliva, feces, urine, etc. and use the same to treat various types of cancer.

In current clinical practice, the diagnosis of cancer is made through history taking, physical examination, and clinical pathology tests, and once suspected, X-ray and endoscopic examinations are performed and tissue biopsy for confirmation is finally performed. However, the diagnosis of cancer by the current clinical test method is possible only when the number of cancer cells is 1 billion or more and the tumor diameter is 1 cm or more. In these cases, the cancer cells already have metastatic ability, and in half or more of the cases, the cancer has already metastasized. Meanwhile, tumor markers for detecting substances directly or indirectly produced by cancer are used in cancer screening, but they cause confusion due to limitations in accuracy, since up to about half thereof appear normal even in the presence of cancer, and they often appear positive even in the absence of cancer. Furthermore, anticancer agents that are mainly used for the treatment of cancer have a problem in that they exhibit an effect only when the volume of cancer is small.

Accordingly, methods of diagnosing cancer through DNA methylation measurement have recently been proposed. DNA methylation mainly occurs at cytosine of CpG islands in the promoter region of specific genes, and as a result, the binding of transcription factors is hindered, resulting in silencing of specific genes. This is the main mechanism by which the function of a gene is lost *in vivo* even in the absence of mutation in the protein-coding sequence of the gene, and is interpreted as the cause of the loss of function of many tumor suppressor genes in human cancer. Although there is controversy over whether methylation of promoter CpG islands directly causes carcinogenesis or is a secondary change leading to carcinogenesis, such abnormal methylation/demethylation in CpG islands has been reported in various cancer cells, including prostate cancer, colon cancer, uterine cancer, and breast cancer. Therefore, this abnormal methylation can be used in various ways, such as early diagnosis of cancer, prediction of carcinogenic risk, prediction of cancer prognosis, follow-up investigation after treatment, and prediction of response to anticancer therapy. Recently, there have been active attempts to use this abnormal methylation for cancer diagnosis and screening by investigating the abnormal methylation using methods such as methylation-specific PCR (hereinafter referred to as MSP), automated base analysis, or bisulfite pyrosequencing. However, most of these methods are limited to detecting and analyzing methylation of a small number of specific genes or promoter regions (e.g., Korean Patent No. 1557183, Korean Patent No. 1191947, and there are limitations in the efficiency and accuracy of diagnosis.

Accordingly, the present inventors have made extensive efforts to solve the above-described problems and develop DNA methylation markers for lung cancer diagnosis with high sensitivity and accuracy, and as a result, have selected methylated DNA regions were selected from TCGA methylation data of lung cancer tissue samples and from tissues and cfDNA of lung cancer patients, and selected lung cancer-specific DNA methylation markers of common regions from the data set, and have found that, when the DNA methylation markers are analyzed, it is possible to lung cancer early with high accuracy, thereby completing the present invention.

## Summary of the Invention

An object of the present invention is to provide a combination of DNA methylation markers for diagnosing lung cancer.

Another object of the present invention is to provide a method for providing information for diagnosing lung cancer using the combination of DNA methylation markers.

Still another object of the present invention is to provide a method for diagnosing lung cancer using the combination of DNA methylation markers.

Yet another object of the present invention is to provide a probe composition and a primer composition, which are capable of detecting the combination of DNA methylation markers, and a kit for diagnosing lung cancer comprising the composition.

To achieve the above objects, the present invention provides a combination of DNA methylation markers for diagnosing lung cancer, comprising DNA methylation markers shown in Table 1.

The present invention also provides a method for providing information for diagnosing lung cancer, comprising steps of: (a) isolating DNA from a biological sample; (b) detecting the methylation level of the combination of DNA methylation markers; and (c) determining the presence of lung cancer if the detected DNA methylation marker level exceeds a cut-off value.

The present invention also provides a method for diagnosing lung cancer, comprising steps of: (a) isolating DNA from a biological sample; (b) detecting the methylation level of the combination of DNA methylation markers; and (c) determining the presence of lung cancer if the detected DNA methylation marker level exceeds a cut-off value.

The present invention also provides a composition for diagnosing lung cancer, comprising a combination of primers capable of amplifying each DNA methylation marker of the combination of DNA methylation markers.

The present invention also provides a composition for diagnosing lung cancer, comprising a combination of probes capable of specifically hybridizing to either a polynucleotide comprising at least 10 or more contiguous nucleotides, which contains a methylated base of a DNA methylation marker of the combination of DNA methylation markers, or a polynucleotide complementary thereto.

The present invention also provides a kit for diagnosing lung cancer, comprising the composition.

## Brief Description of Drawings

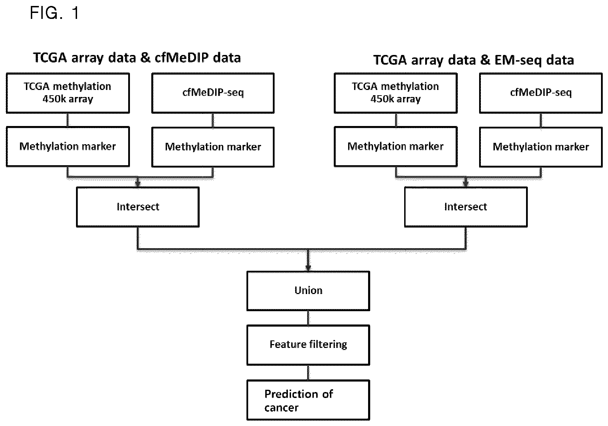

FIG. 1 is a flowchart showing the process of selecting DNA methylation markers for diagnosing lung cancers according to the present invention.

FIG. 2 is an ROC curve showing the lung cancer diagnostic performance of 366 lung cancer-specific DNA methylation markers selected according to one example of the present invention.

FIG. 3 depicts graphs showing the results of evaluating the performance of 366 lung cancer-specific DNA methylation markers selected according to one example of the present invention.

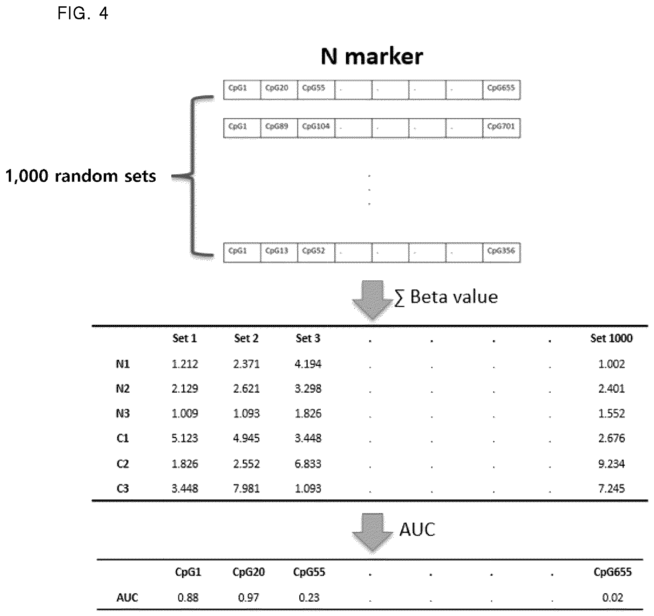

FIG. 4 is a flowchart showing the process of selecting a minimal combination of DNA methylation markers for diagnosing lung cancer according to the present invention.

FIG. 5 is graphs showing the difference in AUC values between lung cancer-specific DNA methylation markers selected according to one example of the present invention and other marker set candidates. Specifically, (A) shows the result for a set of 10 hypermethylated markers, (B) shows the result for a set of 10 hypomethylated markers, (C) shows the result for a set of 7 hypermethylated markers, and (D) shows the results for a set of 7 hypomethylated markers.

FIG. 6 is a ROC_AUC graph showing the results of determining the presence or absence of lung cancer using a minimal combination of lung cancer-specific DNA methylation markers selected according to one example of the present invention.

FIG. 7 shows the results of measuring the difference in methylation level between lung cancer tissue and normal tissue for each of lung cancer-specific DNA methylation markers selected according to one example of the present invention.

FIG. 8 is an ROC_AUC graph showing the results of determining the presence or absence of lung cancer in clinical samples using a combination of 366 lung cancer-specific DNA methylation markers selected according to one example of the present invention.

FIG. 9 is graphs showing the results of evaluating the performance of 366 lung cancer-specific DNA methylation markers, selected according to one example of the present invention, in clinical samples.

FIG. 10 is an ROC_AUC graph showing the results of determining the presence or absence of lung cancer in clinical samples using a combination of 20 lung cancer-specific DNA methylation markers selected according to one example of the present invention.

FIG. 11 is graphs showing the results of evaluating the performance of 20 lung cancer-specific DNA methylation markers, selected according to one example of the present invention, in clinical samples.

FIG. 12 is an ROC_AUC graph showing the results of determining the presence or absence of lung cancer in clinical samples using a combination of 14 lung cancer-specific DNA methylation markers selected according to one example of the present invention.

FIG. 13 is graphs showing the results of evaluating the performance of 14 lung cancer-specific DNA methylation markers, selected according to one example of the present invention, in clinical samples.

**Detailed Description and Preferred Embodiments of the Invention**

Unless otherwise defined, all technical and scientific terms used in the present specification have the same meanings as commonly understood by those skilled in the art to which the present disclosure pertains. In general, the nomenclature used in the present specification is well known and commonly used in the art.

In the present invention, it was sought to develop a model capable of diagnosing lung cancer using methylation information of cell-free nucleic acid in blood and confirm the accuracy thereof.

In the present invention, DNA methylation markers capable of determining the presence or absence of lung cancer were selected by combining methylation data of lung cancer tissue samples described in the TCGA database and methylation data of cell-free nucleic acids extracted from tissue and blood samples of lung cancer patients.

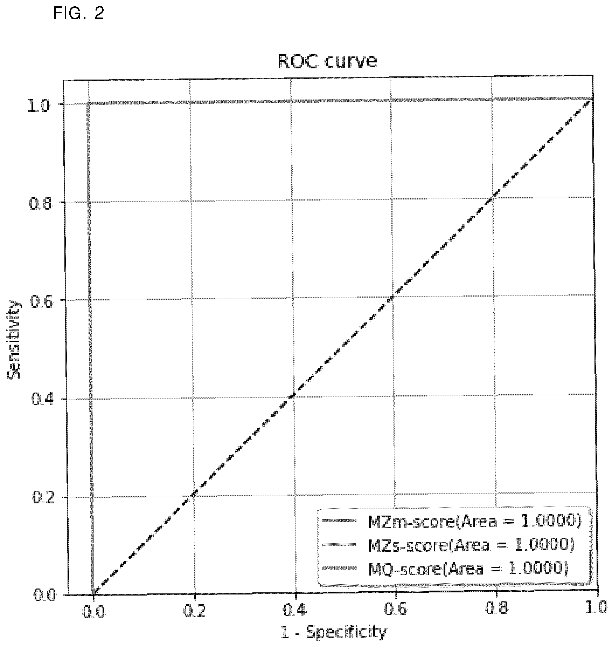

That is, in one example of the present invention, lung cancer-specific methylation regions were selected based on the methylation data of lung cancer tissue samples and normal samples described in the TCGA database, and methylated DNAs extracted from the tissues and blood of lung cancer patients and normal people were sequenced by two methods (cfMeDIP-seq, and EM-seq), and then compared to select lung cancer tissue-specific methylation regions. Overlapping regions between the methylation regions selected from the TCGA database and the methylation regions selected from the lung cancer patient tissue and normal tissue were selected, and overlapping regions between the methylation regions selected from the TCGA database and the methylation regions selected from the blood of lung cancer patients and the blood of normal people were selected. Overlapping regions were first selected from all data sets, and then filtered to select final markers. It was confirmed that, when the presence or absence of lung cancer was determined using the markers, it was possible to determine the presence or absence of lung cancer with high accuracy (FIG. 2).

Therefore, in one aspect, the present invention relates to a combination of DNA methylation markers for diagnosing lung cancer, comprising two or more DNA methylation markers selected from a group consisting of DNA methylation markers shown in Table 1 below.

[Table 1]

| Chromosome | Start position | End position |
|------------|----------------|--------------|
| chr1 | 79472407 | 79472408 |
| chr1 | 79472360 | 79472361 |

(continued)

| Chromosome | Start position | End position |
|---|---|---|
| chr1 | 156357838 | 156357839 |
| chr3 | 147128156 | 147128157 |
| chr3 | 147130535 | 147130536 |
| chr7 | 27225542 | 27225543 |
| chr14 | 57265874 | 57265875 |
| chr16 | 87958492 | 87958493 |
| chr1 | 9896308 | 9896309 |
| chr2 | 10666712 | 10666713 |
| chr7 | 157544911 | 157544912 |
| chr8 | 978225 | 978226 |
| chr11 | 2033842 | 2033843 |
| chr19 | 57175042 | 57175043 |

In the present invention, the combination of DNA methylation markers for lung cancer diagnosis may further comprise DNA markers shown in Table 2 below, without being limited thereto.

[Table 2]

| Chromosome | Start position | End posiiton |
|---|---|---|
| chr1 | 157164795 | 157164796 |
| chr3 | 181442675 | 181442676 |
| chr5 | 92909428 | 92909429 |
| chr7 | 1303350 | 1303351 |
| chr12 | 1943888 | 1943889 |
| chr17 | 79652243 | 79652244 |

In the present invention, the combination of DNA methylation markers for lung cancer diagnosis may further comprise two or more DNA methylation markers selected from the group consisting of DNA markers shown in Table 3 below, without being limited thereto.

[Table 3]

| Chromosome | Start position | End position | chromosome | Start position | End position |
|---|---|---|---|---|---|
| chr1 | 38412711 | 38412712 | chr12 | 132847907 | 132847908 |
| chr1 | 63790044 | 63790045 | chr12 | 132892613 | 132892614 |
| chr1 | 75595970 | 75595971 | chr12 | 133028690 | 133028691 |
| chr1 | 79472282 | 79472283 | chr13 | 109741117 | 109741118 |
| chr1 | 79472452 | 79472453 | chr13 | 109741176 | 109741177 |
| chr1 | 110611218 | 110611219 | chr13 | 112200864 | 112200865 |
| chr1 | 119526060 | 119526061 | chr13 | 112607846 | 112607847 |
| chr1 | 119529219 | 119529220 | chr13 | 114056351 | 114056352 |
| chr1 | 119532093 | 119532094 | chr13 | 114065008 | 114065009 |
| chr1 | 119535928 | 119535929 | chr13 | 114065669 | 114065670 |
| chr1 | 119548825 | 119548826 | chr13 | 114311898 | 114311899 |

(continued)

| Chromosome | Start position | End position | chromosome | Start position | End position |
|---|---|---|---|---|---|
| chr1 | 170630070 | 170630071 | chr14 | 77328166 | 77328167 |
| chr1 | 214153294 | 214153295 | chr14 | 100658902 | 100658903 |
| chr1 | 214153460 | 214153461 | chr14 | 101334582 | 101334583 |
| chr1 | 214153472 | 214153473 | chr14 | 104648347 | 104648348 |
| chr10 | 81003175 | 81003176 | chr14 | 104837541 | 104837542 |
| chr10 | 103043991 | 103043992 | chr14 | 104837550 | 104837551 |
| chr10 | 118892211 | 118892212 | chr14 | 105031243 | 105031244 |
| chr11 | 31819444 | 31819445 | chr14 | 106174668 | 106174669 |
| chr12 | 54339653 | 54339654 | chr14 | 106239286 | 106239287 |
| chr12 | 85667616 | 85667617 | chr14 | 106320669 | 106320670 |
| chr12 | 85671811 | 85671812 | chr15 | 26235162 | 26235163 |
| chr12 | 85673221 | 85673222 | chr15 | 78505051 | 78505052 |
| chr13 | 79169872 | 79169873 | chr15 | 89386800 | 89386801 |
| chr13 | 112726118 | 112726119 | chr16 | 605214 | 605215 |
| chr13 | 112759719 | 112759720 | chr16 | 605347 | 605348 |
| chr14 | 29235193 | 29235194 | chr16 | 644081 | 644082 |
| chr14 | 29235196 | 29235197 | chr16 | 1052939 | 1052940 |
| chr14 | 29254942 | 29254943 | chr16 | 1061053 | 1061054 |
| chr14 | 57278188 | 57278189 | chr16 | 1093823 | 1093824 |
| chr14 | 99729088 | 99729089 | chr16 | 1147829 | 1147830 |
| chr14 | 101923256 | 101923257 | chr16 | 1233872 | 1233873 |
| chr16 | 66613266 | 66613267 | chr16 | 1256295 | 1256296 |
| chr16 | 86547530 | 86547531 | chr16 | 1257884 | 1257885 |
| chr19 | 11785188 | 11785189 | chr16 | 1271741 | 1271742 |
| chr19 | 12624466 | 12624467 | chr16 | 1271998 | 1271999 |
| chr19 | 22806184 | 22806185 | chr16 | 1316081 | 1316082 |
| chr19 | 58220494 | 58220495 | chr16 | 2213440 | 2213441 |
| chr19 | 58220516 | 58220517 | chr16 | 2213749 | 2213750 |
| chr19 | 58220657 | 58220658 | chr16 | 11770950 | 11770951 |
| chr19 | 58220662 | 58220663 | chr16 | 22326535 | 22326536 |
| chr19 | 58220773 | 58220774 | chr16 | 81564192 | 81564193 |
| chr2 | 45159894 | 45159895 | chr16 | 85019773 | 85019774 |
| chr2 | 63281844 | 63281845 | chr16 | 85678884 | 85678885 |
| chr2 | 66666684 | 66666685 | chr16 | 86465884 | 86465885 |
| chr2 | 73148229 | 73148230 | chr16 | 87682572 | 87682573 |
| chr2 | 74875227 | 74875228 | chr16 | 87958407 | 87958408 |
| chr2 | 119599459 | 119599460 | chr16 | 88442086 | 88442087 |
| chr2 | 119607379 | 119607380 | chr16 | 88460610 | 88460611 |
| chr2 | 119607573 | 119607574 | chr16 | 89016957 | 89016958 |

(continued)

| Chromosome | Start position | End position | chromosome | Start position | End position |
|---|---|---|---|---|---|
| chr2 | 171676809 | 171676810 | chr16 | 89029055 | 89029056 |
| chr2 | 172952948 | 172952949 | chr16 | 89110004 | 89110005 |
| chr2 | 176948728 | 176948729 | chr16 | 89118838 | 89118839 |
| chr2 | 176981336 | 176981337 | chr16 | 89245231 | 89245232 |
| chr2 | 176986460 | 176986461 | chr16 | 89704682 | 89704683 |
| chr2 | 176993017 | 176993018 | chr17 | 38860042 | 38860043 |
| chr2 | 177012191 | 177012192 | chr17 | 40705588 | 40705589 |
| chr2 | 177014996 | 177014997 | chr17 | 48680554 | 48680555 |
| chr2 | 177015044 | 177015045 | chr17 | 49027095 | 49027096 |
| chr2 | 177027043 | 177027044 | chr17 | 77174120 | 77174121 |
| chr2 | 177029073 | 177029074 | chr17 | 79096529 | 79096530 |
| chr2 | 177029459 | 177029460 | chr17 | 79099882 | 79099883 |
| chr2 | 177029608 | 177029609 | chr17 | 79961522 | 79961523 |
| chr2 | 182543233 | 182543234 | chr17 | 80342459 | 80342460 |
| chr20 | 43726765 | 43726766 | chr18 | 44259973 | 44259974 |
| chr3 | 138662315 | 138662316 | chr18 | 61817320 | 61817321 |
| chr3 | 147111660 | 147111661 | chr18 | 76485587 | 76485588 |
| chr3 | 147113918 | 147113919 | chr19 | 3936724 | 3936725 |
| chr3 | 147114406 | 147114407 | chr19 | 4552496 | 4552497 |
| chr3 | 147142182 | 147142183 | chr19 | 13394116 | 13394117 |
| chr3 | 157812226 | 157812227 | chr19 | 18085190 | 18085191 |
| chr3 | 157813327 | 157813328 | chr2 | 1133947 | 1133948 |
| chr3 | 157820591 | 157820592 | chr2 | 1880012 | 1880013 |
| chr3 | 157821262 | 157821263 | chr2 | 4931004 | 4931005 |
| chr3 | 157821994 | 157821995 | chr2 | 4931074 | 4931075 |
| chr4 | 11370466 | 11370467 | chr2 | 10638113 | 10638114 |
| chr4 | 15704680 | 15704681 | chr2 | 26800399 | 26800400 |
| chr4 | 41875470 | 41875471 | chr2 | 233198590 | 233198591 |
| chr4 | 85402870 | 85402871 | chr2 | 239544973 | 239544974 |
| chr4 | 111543401 | 111543402 | chr2 | 241083864 | 241083865 |
| chr4 | 111552040 | 111552041 | chr20 | 5451900 | 5451901 |
| chr4 | 174452835 | 174452836 | chr20 | 60120472 | 60120473 |
| chr5 | 42952113 | 42952114 | chr20 | 60456498 | 60456499 |
| chr5 | 54519023 | 54519024 | chr20 | 60456503 | 60456504 |
| chr5 | 76932016 | 76932017 | chr20 | 61625364 | 61625365 |
| chr5 | 76934327 | 76934328 | chr20 | 61695598 | 61695599 |
| chr5 | 140536238 | 140536239 | chr20 | 61755045 | 61755046 |
| chr5 | 172671754 | 172671755 | chr20 | 61792443 | 61792444 |
| chr5 | 172672766 | 172672767 | chr20 | 62079911 | 62079912 |

(continued)

| Chromosome | Start position | End position | chromosome | Start position | End position |
|---|---|---|---|---|---|
| chr6 | 10425648 | 10425649 | chr21 | 46420451 | 46420452 |
| chr6 | 27648004 | 27648005 | chr21 | 46677879 | 46677880 |
| chr6 | 50804147 | 50804148 | chr22 | 29704483 | 29704484 |
| chr6 | 100050791 | 100050792 | chr22 | 37769171 | 37769172 |
| chr6 | 100066698 | 100066699 | chr22 | 38506737 | 38506738 |
| chr6 | 100903817 | 100903818 | chr22 | 43835600 | 43835601 |
| chr6 | 100917397 | 100917398 | chr22 | 44708861 | 44708862 |
| chr6 | 105388731 | 105388732 | chr22 | 49137906 | 49137907 |
| chr6 | 134210946 | 134210947 | chr22 | 49376886 | 49376887 |
| chr7 | 8482235 | 8482236 | chr22 | 49487343 | 49487344 |
| chr7 | 8482325 | 8482326 | chr22 | 50470101 | 50470102 |
| chr7 | 25898688 | 25898689 | chr3 | 14615134 | 14615135 |
| chr7 | 26897202 | 26897203 | chr3 | 128182392 | 128182393 |
| chr7 | 26897253 | 26897254 | chr3 | 134146405 | 134146406 |
| chr7 | 27192056 | 27192057 | chr3 | 194078147 | 194078148 |
| chr7 | 27205217 | 27205218 | chr3 | 196065306 | 196065307 |
| chr7 | 27206073 | 27206074 | chr3 | 196065318 | 196065319 |
| chr7 | 27225772 | 27225773 | chr3 | 196065320 | 196065321 |
| chr7 | 39649443 | 39649444 | chr3 | 196065328 | 196065329 |
| chr7 | 96631680 | 96631681 | chr3 | 196065569 | 196065570 |
| chr7 | 96636616 | 96636617 | chr4 | 1538525 | 1538526 |
| chr7 | 96642794 | 96642795 | chr5 | 476497 | 476498 |
| chr7 | 129423053 | 129423054 | chr5 | 476646 | 476647 |
| chr7 | 153584582 | 153584583 | chr5 | 555251 | 555252 |
| chr7 | 153584597 | 153584598 | chr5 | 1003897 | 1003898 |
| chr8 | 4849522 | 4849523 | chr5 | 1119025 | 1119026 |
| chr8 | 65498812 | 65498813 | chr5 | 1140096 | 1140097 |
| chr9 | 79638117 | 79638118 | chr5 | 1202449 | 1202450 |
| chr1 | 942161 | 942162 | chr5 | 1219972 | 1219973 |
| chr1 | 1267193 | 1267194 | chr5 | 1232590 | 1232591 |
| chr1 | 1267201 | 1267202 | chr5 | 1244729 | 1244730 |
| chr1 | 2345333 | 2345334 | chr5 | 1257084 | 1257085 |
| chr1 | 2750406 | 2750407 | chr5 | 1767994 | 1767995 |
| chr1 | 2764669 | 2764670 | chr5 | 2103257 | 2103258 |
| chr1 | 2847632 | 2847633 | chr5 | 2132336 | 2132337 |
| chr1 | 2850512 | 2850513 | chr5 | 17654594 | 17654595 |
| chr1 | 2904595 | 2904596 | chr5 | 169531595 | 169531596 |
| chr1 | 2936277 | 2936278 | chr5 | 180046901 | 180046902 |
| chr1 | 2949633 | 2949634 | chr5 | 180047184 | 180047185 |

(continued)

| Chromosome | Start position | End position | chromosome | Start position | End position |
|---|---|---|---|---|---|
| chr1 | 3058151 | 3058152 | chr6 | 466893 | 466894 |
| chr1 | 3083061 | 3083062 | chr6 | 25218752 | 25218753 |
| chr1 | 3150935 | 3150936 | chr6 | 169740781 | 169740782 |
| chr1 | 3210325 | 3210326 | chr7 | 1403804 | 1403805 |
| chr1 | 3495611 | 3495612 | chr7 | 4841835 | 4841836 |
| chr1 | 3606892 | 3606893 | chr7 | 71177239 | 71177240 |
| chr1 | 22925489 | 22925490 | chr7 | 154542060 | 154542061 |
| chr1 | 35220814 | 35220815 | chr7 | 155191845 | 155191846 |
| chr1 | 56880606 | 56880607 | chr7 | 157085979 | 157085980 |
| chr10 | 44197967 | 44197968 | chr7 | 157502455 | 157502456 |
| chr10 | 134652276 | 134652277 | chr7 | 157776887 | 157776888 |
| chr10 | 134833372 | 134833373 | chr7 | 157968578 | 157968579 |
| chr10 | 134833874 | 134833875 | chr7 | 158030726 | 158030727 |
| chr10 | 134838656 | 134838657 | chr7 | 158331217 | 158331218 |
| chr10 | 134842688 | 134842689 | chr7 | 158799715 | 158799716 |
| chr10 | 134912012 | 134912013 | chr7 | 158820591 | 158820592 |
| chr10 | 134928457 | 134928458 | chr8 | 1140682 | 1140683 |
| chr10 | 134929181 | 134929182 | chr8 | 10753237 | 10753238 |
| chr10 | 135015300 | 135015301 | chr8 | 141107113 | 141107114 |
| chr10 | 135016451 | 135016452 | chr8 | 142517494 | 142517495 |
| chr10 | 135018765 | 135018766 | chr8 | 142841913 | 142841914 |
| chr10 | 135018844 | 135018845 | chr8 | 142841965 | 142841966 |
| chr11 | 393606 | 393607 | chr8 | 143201145 | 143201146 |
| chr11 | 1006121 | 1006122 | chr8 | 143208101 | 143208102 |
| chr11 | 1050848 | 1050849 | chr8 | 143262200 | 143262201 |
| chr11 | 1257516 | 1257517 | chr8 | 143333654 | 143333655 |
| chr11 | 1464300 | 1464301 | chr8 | 143509263 | 143509264 |
| chr11 | 2182540 | 2182541 | chr8 | 143535735 | 143535736 |
| chr11 | 47359115 | 47359116 | chr8 | 143605535 | 143605536 |
| chr11 | 61302186 | 61302187 | chr8 | 143625791 | 143625792 |
| chr11 | 66104485 | 66104486 | chr8 | 143915919 | 143915920 |
| chr11 | 68964113 | 68964114 | chr8 | 143915954 | 143915955 |
| chr11 | 69192623 | 69192624 | chr8 | 143961145 | 143961146 |
| chr11 | 69192697 | 69192698 | chr8 | 144203483 | 144203484 |
| chr11 | 70781118 | 70781119 | chr8 | 144366016 | 144366017 |
| chr11 | 120008998 | 120008999 | chr8 | 144416327 | 144416328 |
| chr11 | 128812875 | 128812876 | chr8 | 144801223 | 144801224 |
| chr12 | 2613914 | 2613915 | chr8 | 145697495 | 145697496 |
| chr12 | 29650795 | 29650796 | chr8 | 145758486 | 145758487 |

(continued)

| Chromosome | Start position | End position | chromosome | Start position | End position |
|---|---|---|---|---|---|
| chr12 | 94954828 | 94954829 | chr9 | 137718814 | 137718815 |
| chr12 | 124693173 | 124693174 | chr9 | 138018566 | 138018567 |
| chr12 | 131580615 | 131580616 | chr9 | 140041732 | 140041733 |
| chr12 | 132680972 | 132680973 | chr9 | 140333139 | 140333140 |
| chr12 | 132839436 | 132839437 | | | |

The term "DNA methylation" in the present invention refers to covalent bonding of a methyl group to the C5 position of a cytosine base in genomic DNA. The methylation level means, for example, the level of methylation present in a DNA base sequence in all genomic regions and some non-genomic regions, and in the present invention, it means the degree of methylation of the DNA methylation marker. In the DNA methylation marker, methylation may occur throughout the entire sequence or part of the sequence.

In the present invention, the lung cancer refers to a malignant tumor that occurs in the lungs, and more specifically, it refers to primary lung cancer originating from tissues (bronchial tubes, bronchioles, alveoli, etc.) that constitute the lungs and metastatic lung cancer that occurs in other organs and metastasizes to the lungs. Primary lung cancer may be divided into non-small cell carcinoma and small cell carcinoma, and non-small cell lung carcinoma may be further divided into squamous cell carcinoma, adenocarcinoma, and large-cell carcinoma.

In another aspect, the present invention relates to a method for providing information for diagnosing lung cancer, comprising steps of:

(a) isolating DNA from a biological sample;
(b) detecting the methylation level of the combination of DNA methylation markers; and
(c) determining the presence of lung cancer if the detected DNA methylation marker level exceeds a cut-off value.

In another aspect, the present invention relates to a method for diagnosing lung cancer, comprising steps of:

(a) isolating DNA from a biological sample;
(b) detecting the methylation level of the combination of DNA methylation markers; and
(c) determining the presence of lung cancer if the detected DNA methylation marker level exceeds a cut-off value.

In the present invention, the DNA may be, without limitation, a DNA extracted from a biological sample, but preferably may be a fragment of cell-free nucleic acid or intracellular nucleic acid, without being limited thereto.

In the present invention, the biological sample means any material, biological fluid, tissue or cells obtained from or derived from a subject, and examples thereof include, but are not limited to, whole blood, leukocytes, peripheral blood mononuclear cells, leukocyte buffy coat, blood including plasma and serum, sputum, tears, mucus, nasal washes, nasal aspirate, breath, urine, semen, saliva, peritoneal washings, pelvic fluids, cystic fluid, meningeal fluid, amniotic fluid, glandular fluid, pancreatic fluid, lymph fluid, pleural fluid, nipple aspirate, bronchial aspirate, synovial fluid, joint aspirate, organ secretions, cells, cell extract, semen, hair, saliva, urine, oral cells, placental cells, cerebrospinal fluid, and mixtures thereof.

In the present invention, detecting the methylation level in step (b) may be performed by various known methods, and is preferably performed by bisulfite conversion or methylated DNA immunoprecipitation (MeDIP), without being limited thereto.

In the present invention, methods capable of detecting DNA methylation further include a restriction enzyme-based detection method, which is a method in which an unmethylated nucleic acid is cleaved using methylation restriction enzymes (MREs) or a specific sequence (recognition site) is cleaved regardless of methylation, and is analyzed using a hybridization method or PCR.

In the present invention, bisulfite conversion-based methods include whole-genome bisulfite sequencing (WGBS), reduced-representation bisulfite sequencing (RRBS), methylated CpG tandems amplification and sequencing (MCTA-seq), targeted bisulfite sequencing, methylation array, and methylation-specific PCR (MSP).

In the present invention, methods for enriching and analyzing methylated DNA include methylated DNA immunoprecipitation sequencing (MeDIP-seq), methyl-CpG binding domain protein capture sequencing (MBD-seq), and the like.

In the present invention, another method capable of analyzing methylated DNA is 5-hydroxymethylation profiling, examples of which include 5hmC-Seal (hMe-Seal), hmC-CATCH, hydroxymethylated DNA immunoprecipitation sequencing (hMeDIP-seq), and oxidative bisulfite conversion.

In the present invention, detecting the methylation level in step (b) may be performed using any one method selected from the group consisting of PCR, methylation-specific PCR, real time methylation-specific PCR, PCR using methylated DNA-specific binding protein, quantitative PCR, PCR using methylation-specific PNA, melting curve analysis, DNA chip assay, pyrosequencing, bisulfite sequencing, and methylation next-generation sequencing, without being limited thereto.

In the present invention, the next-generation sequencer may be used for any sequencing method known in the art. Sequencing of the nucleic acids isolated by the selection method is typically performed using next-generation sequencing (NGS). Next-generation sequencing includes any sequencing method that determines the nucleotide sequence of either individual nucleic acid molecules or clonally expanded proxies for individual nucleic acid molecules in a high throughput fashion (e.g., $10^5$ or more molecules are sequenced simultaneously). In one embodiment, the relative abundance of the nucleic acid species in the library may be estimated by counting the relative number of occurrences of their cognate sequences in the data generated by the sequencing experiment. Next generation sequencing methods are known in the art, and are described, e.g., in Metzker, M. (2010) Nature Biotechnology Reviews 11:31-46, which is incorporated herein by reference.

In one embodiment, the next-generation sequencing allows for the determination of the nucleotide sequence of an individual nucleic acid molecule (e.g., Helicos BioSciences' HeliScope Gene Sequencing system, and Pacific Biosciences' PacBio RS system). In other embodiments, the sequencing method determines the nucleotide sequence of clonally expanded proxies for individual nucleic acid molecules (e.g., the Solexa sequencer, Illumina Inc., San Diego, Calif.; 454 Life Sciences (Branford, Conn.), and Ion Torrent), e.g., massively parallel short-read sequencing (e.g., the Solexa sequencer, Illumina Inc., San Diego, Calif.), which generates more bases of sequence per sequencing unit than other sequencing methods that generate fewer but longer reads. Other methods or machines for next-generation sequencing include, but are not limited to, the sequencers provided by 454 Life Sciences (Branford, Conn.), Applied Biosystems (Foster City, Calif.; SOLiD sequencer), Helicos BioSciences Corporation (Cambridge, Mass.), and emulsion and microfluidic sequencing technology nanodroplets (e.g., GnuBio droplets).

Platforms for next-generation sequencing include, but are not limited to, Roche/454's Genome Sequencer (GS) FLX System, Illumina/Solexa's Genome Analyzer (GA), Life/APG's Support Oligonucleotide Ligation Detection (SOLiD) system, Polonator's G.007 system, Helicos BioSciences' HeliScope Gene Sequencing system, Oxford Nanopore Technologies' PromethION, GriION, MinION system, and Pacific Biosciences' PacBio RS system.

In the present invention, the methylation level in step (b) may be expressed as a beta value, without being limited thereto.

In the present invention, step (c) may be performed by a method comprising the following steps:

(c-i) calculating the standard deviation and mean of beta values obtained from normal group samples;
(c-ii) calculating a z-score using the following equation 1;

Equation 1:

$$Z\ score = \frac{methylation\ level\ in\ step\ (b) - mean\ of\ methylation\ levels\ in\ normal\ samples}{standard\ deviation\ of\ methylation\ levels\ in\ normal\ samples}$$

(c-ii) calculating the sum, mean, or standard deviation of the calculated z-scores; and
(c-iii) determining the presence of lung cancer if the sum, mean, or standard deviation of the calculated z scores exceeds a cut-off value.

In the present invention, step (c) may be performed by a step of comparing information on the detected methylation level of the combination of DNA methylation markers with the value of the normal samples and determining the presence of lung cancer if the detected methylation level is greater than the cut-off value, without being limited thereto.

In the present invention, the cut-off value in step (c) may be, without limitation, a value that may determine the presence or absence of lung cancer, and preferably, it may be 99% to 75%, more preferably 97% to 80%, most preferably 95%, of the methylation level of the normal samples, without being limited thereto.

In the present invention, in step (c), the information on the detected methylation level of the combination of DNA methylation markers may be one or more values selected from the group consisting of the sum, difference, product, mean, log of product, log of sum, median, quantile, minimum, maximum, variance, standard deviation, absolute deviation, coefficient of variation, reciprocal values thereof, and combinations thereof, of the beta values of the individual markers, without being limited thereto.

In the present invention, as is well known to those skilled in the art, when calculating the information on the methylation level as the beta value, the beta value of the hypermethylated methylation marker is used as is, and the beta value of the hypomethylated methylation marker is calculated by subtracting from a certain cut-off value such as 100 or 1, or by multiplying by -1.

In the present invention, the cut-off value in step (c) may be, without limitation, a value that may determine the presence or absence of lung cancer. Preferably, the cut-off value of the mean of the z-scores may be a value between 0.2 and 0.9, more preferably between 0.3 and 0.8, most preferably between 0.5 and 0.6, the cut-off value of the sum of the z-scores may be a value between 100 and 300, more preferably between 150 and 200, most preferably between 180 and 200, and the cut-off value of the standard deviation of the z-scores may be a value between 0.5 and 2, more preferably between 0.8 and 1.5, most preferably between 0.9 and 1.2, without being limited thereto.

In another aspect, the present invention relates to

a composition for diagnosing lung cancer, comprising a combination of primers capable of amplifying each DNA methylation marker of the combination of DNA methylation markers.

In the present invention, the appropriate length of the primer may vary depending on the intended use, but may generally consist of 15 to 30 nucleotides. The primer sequence does not need to be completely complementary to the template, but should be sufficiently complementary to hybridize with the template. The primer is capable of hybridizing to a DNA sequence including a methylation marker and amplifying a DNA fragment including the methylation marker. The primer of the present invention may be used in a diagnostic kit or prediction method for determining the presence or absence of lung cancer by detecting the level of DNA methylation.

In the present invention, the primer capable of amplifying the DNA methylation marker may be, without limitation, a nucleotide sequence of the same chromosome that does not directly include the marker region, but specifically, it may include 1 to 1,000-bp 5' upstream and 1 to 1,000-bp 3' downstream of the marker region, and more specifically, it may include 1 to 200-bp 5' upstream and 1 to 200-bp 3' downstream of the marker region, without being limited thereto.

In another aspect, the present invention relates to

a composition for diagnosing lung cancer, comprising a combination of probes capable of specifically hybridizing to either a polynucleotide comprising at least 10 or more contiguous nucleotides, which contains a methylated base of a DNA methylation marker of the combination of DNA methylation markers, or a polynucleotide complementary thereto.

In the present invention, the probe may be methylation-specific, which means that it specifically hybridizes only to a methylated nucleic acid of the methylation marker region. Here, hybridization is usually performed under stringent conditions, for example, a salt concentration of 1 M or less and a temperature of 25°C or higher. For example, conditions of 5X SSPE (750 mM NaCl, 50 mM Na Phosphate, 5 mM EDTA, pH 7.4) and 25 to 30°C may be suitable for methylation-specific probe hybridization.

In the present invention, the probe refers to a hybridization probe, which refers to an oligonucleotide capable of binding sequence-specifically to a complementary strand of a nucleic acid. The methylation-specific probe of the present invention may hybridize to a DNA fragment derived from one individual but does not hybridize to a fragment derived from another individual, because methylation exists in nucleic acid fragments derived from two individuals of the same species. In this case, hybridization conditions should be sufficiently stringent that there is a significant difference in hybridization intensity so that hybridization occurs depending on whether methylation is present. It is preferable that the central portion of the probe of the present invention be aligned to the region of the methylation marker. The probe of the present invention may be used in a diagnostic kit or prediction method for determining the presence or absence of lung cancer by detecting the level of DNA methylation.

In another aspect, the present invention relates to

a kit for diagnosing lung cancer, comprising any one of the above-described compositions.

In the present invention, the kit may comprise not only the polynucleotide of the present invention, but also one or more other component compositions, solutions or devices suitable for analytical methods. In one embodiment, the kit of the present invention may be a kit comprising essential elements necessary for performing PCR, and may further comprise a test tube or other appropriate container, a reaction buffer (various pHs and magnesium concentrations), deoxynucleotides (dNTPs), enzymes such as Taq polymerase and reverse transcriptase, DNase, RNase inhibitor, DEPC-water, and sterile water. In another aspect, the kit of the present invention may be a kit for predicting blood statin concentration, comprising essential elements necessary for performing DNA chip assay. The DNA chip kit may comprise a substrate to which the methylation-specific polynucleotide, primer or probe is attached, wherein the substrate may comprise a nucleic acid corresponding to a quantitative control gene or a fragment thereof.

## Examples

Hereinafter, the present invention will be described in more detail by way of examples. These examples are only intended to illustrate the present invention, and it will be apparent to those skilled in the art that the scope of the present invention is not to be construed as being limited by these examples.

## Example 1. Selection of Lung Cancer-Specific Methylation Regions from TCGA Methylation 450K Array Data

The degree of methylation was determined using Infinium Human Methylation 450K BeadChip array data (UCSC Xena, http://xena.ucsc.edu) from The Cancer Genome Atlas (TCGA). DNA extracted from tissue is converted by bisulfite treatment, and DNA methylation can be determined through modification of cytosine bases. The degree of methylation can be determined for each region, and the beta value, which represents the degree of methylation, was used to select differentially methylated regions between lung cancer tissue and surrounding normal tissue.

TCGA methylation 450k array data were divided into lung adenocarcinoma and lung squamous cell carcinoma as shown in Table 4 and Table 5 below, and further divided into training and test groups, and marker selection was performed using the training group.

[Table 4]

| LUAD (adenocarcinoma) | Primary Solid Tumor | Solid Tissue Normal | Normal whole blood | Total |
|---|---|---|---|---|
| Training | 320 | 32 | 459 | 811 |
| Test | 138 | - | 197 | 335 |
| Total | 458 | 32 | 656 | 1,146 |

[Table 5]

| LUSC (squamous cell carcinoma) | Primary Solid Tumor | Solid Tissue Normal | Normal whole blood | Total |
|---|---|---|---|---|
| Training | 259 | 42 | 459 | 760 |
| Test | 111 | - | 197 | 308 |
| Total | 370 | 42 | 656 | 1,068 |

First, missing values were excluded from about 480k regions.

Then, to select lung adenocarcinoma-specific methylation regions, Limma (Linear Models for Microarray Data) software was used to select regions with an FDR value of less than 0.01 and an absolute delta beta value greater than 0.25, thereby selecting 617 hypomethylated CpGs and 3,114 hypermethylated CpGs specific to lung adenocarcinoma.

To select lung squamous cell carcinoma-specific methylation regions, Limma (Linear Models for Microarray Data) software was used to select regions with an FDR value of less than 0.01 and an absolute delta beta value of greater than 0.25, thereby selecting 8,105 hypomethylated CpGs and 5,486 hypermethylated CpGs specific to lung squamous cell carcinoma.

## Example 2. Next-Generation Sequencing of cfDNA Extracted from Blood (cfMeDIP-Seq)

Blood was collected from 25 lung cancer patients and 190 normal people, and then subjected to a first centrifugation under the conditions of 3,000 rpm, 25°C, and 10 minutes to separate only the plasma portion. Then, the plasma separated by the first centrifugation was centrifuged to a second centrifugation under the conditions of 16,000 g, 25°C, and 10 minutes to separate the plasma supernatant excluding the precipitate. Cell-free DNA was extracted from the separated plasma using the Chemagen DNA kit, and subjected to adaptor ligation using the Truseq Nano DNA HT library prep kit (Illumina), followed by 5mC immunoprecipitation using the antibody of the cfMeDIP kit (Diagnode) at 10 rpm and 4°C for 17 hours. After purification, PCR enrichment was performed using the Truseq Nano DNA HT library prep kit (Illumina) to produce the final library. The produced library was sequenced using Novaseq 6000 (Illumina) in 150 paired-end mode, producing about 100 million reads per sample.

## Example 3. Selection of Lung Cancer-Specific Methylation Regions through cfMeDIP-Seq Data Analysis

Since the methylated cell-free nucleic acid was sequenced in Example 2, the obtained nucleic acid fragment data are in a methylated state and can be aligned to the human reference genome, thereby identifying the methylated regions in the entire human genome. MeDIP-Seq data represent methylated regions, and the normalized values for each 300-bp bin were used to select differentially methylated regions between the lung cancer group and the normal group.

cfMeDIP-Seq data were divided into training and test groups as shown in Table 6 below. Marker selection was performed using the training group, and marker performance was evaluated using the test group.

[Table 6]

|  | Lung cancer | Normal | Total |
|---|---|---|---|
| **Training** | 19 | 152 | 171 |
| **Test** | 6 | 38 | 44 |
| **Total** | 25 | 190 | 215 |

First, adapter trimming and quality trimming of fastq files were performed using Trim Galore (version 0.6.6). Then, nucleic acid fragment data were aligned to the reference genome (hg19) using the BWA (version 0.7.17-r1188) alignment tool, and duplicate PCR fragments were removed using the SAMtools rmdup (version 1.11) tool. Then, nucleic acid fragments with a mapping quality of less than 10 were removed using the SAMtools view (version 1.11) tool. Then, only chr1-22, X, and Y were retained, while the others were discarded. The entire genome was divided into 300-bp bins, and then read counts for each 300-bp bin were calculated.

Blacklist regions (Low_mappability_island, centromeric_repeat, etc.) and bins with a total read count of less than 10 across all samples were excluded.

TMM normalized values for each 300-bp bin were generated using the edgeR (Empirical Analysis of Digital Gene Expression Data in R) software.

Finally, using the edgeR software, 120,176 hypermethylated regions and 185,638 hypomethylated regions specific to lung cancer with an FDR value of less than 0.05 and an absolute value of log2 fold change of more than 2 were selected, and then CpGs included in the selected bins were extracted.

Then, a model was constructed to distinguish between the lung cancer and normal groups by binning 138 hypermethylated CpGs and 1309 hypomethylated CpGs that were found identically in TCGA array data and cfMeDIP-seq data, and the performance of accuracy = 0.98 and AUC = 1.00 was confirmed in the test group.

## Example 4. Enzymatic Methylation Sequencing (EM-seq)

### 4-1. Whole Genome EM-seq

Blood was collected from 7 lung cancer patients and 10 normal people, and then subjected to a first centrifugation under the conditions of 3000 rpm, 25°C, and 10 minutes to separate only the plasma portion. The plasma separated by the first centrifugation was subjected to a second centrifugation time under the conditions of 16,000 g, 25°C, and 10 minutes to separate the plasma. Then, 400 µl of buffy coat was isolated and genomic DNA was extracted therefrom using the QIAmp DNA mini kit (Qiagen, Germany).

In addition, 10 to 30 ng of fresh frozen tissue from lung cancer tissue and surrounding normal tissue of the 7 lung cancer patients was disrupted using FastPrep-24, and then genomic DNA was extracted therefrom using the QIAmp DNA mini kit (Qiagen, Germany).

The concentrations of the genomic DNAs extracted from the tissues and blood was measured using the Qubit dsDNA HS assay kit (Thermo Fisher Scientific, USA), the purity was checked using Nanodrop, and then 50 µl DNA (200 ng) was sheared to 240-290 bp using Covaris. The DNA size was checked using the D1000 screen tape & reagent (Agilent, USA) with Tapestation 4200 (Agilent, USA).

For 200-ng sheared DNA, methylation conversion was performed by replacing unmethylated cytosines with uracil using ten-eleven translocation dioxygenase 2 (TET2) and APOBEC, and then a library was prepared using enzymatic methyl-seq (NEB Kit). The concentration and size of the prepared DNA library were measured using the Qubit DS DNA HS assay kit (Thermo Fisher Scientific, USA) and Tapestation 4200 (Agilent, USA), respectively. Then, sequencing was performed at a final concentration of 2 nM using Novaseq 6000 (Illumina) in 150 paired-end mode, generating approximately 650 million reads per sample.

### 4-2. cfDNA Targeted Methylome Panel-Based EM-Seq

The methylome panel used was Twist Human Methylome Panel (Twist Bioscience, USA).

Blood was collected from 12 lung cancer patients, including 7 lung cancer patients in Example 4-1, and 7 normal people, and then subjected to a first centrifugation under the conditions of 3,000 rpm, 25°C, and 10 minutes to separate only the plasma portion. Then, the plasma isolated by the first centrifugation was subjected to a second centrifugation under the conditions of 16,000 g, 25°C, and 10 minutes to separate the plasma supernatant excluding the precipitate. Cell-free DNA was extracted from the separated plasma using the Mag-bind cfDNA kit, and the concentration was measured using the Qubit DS DNA HS assay kit (Thermo Fisher Scientific, USA). For the maximum amount of the extracted cfDNA, methylation conversion was performed by replacing unmethylated cytosines with uracil using ten-eleven translocation dioxygenase 2 (TET2) and APOBEC, and then a library was prepared using enzymatic methyl-seq (NEB Kit).

The concentration and size of the prepared DNA library were measured using the Qubit DS DNA HS assay kit (Thermo Fisher Scientific, USA) and Tapestation 4200 (Agilent, USA), respectively. 200 ng of the library was pooled into groups of 8 samples, hybridization was performed, and the concentration of the captured sample was measured using the high sensitivity D1000 screen tape and reagent (Agilent, USA) with Tapestation 4200 (Agilent, USA). Sequencing was performed at a final concentration of 2 nM using Novaseq 6000 (Illumina) in 150 paired-end mode, generating approximately 150 million reads per sample.

### Example 5. Selection of Lung Cancer-Specific Methylation Regions through EM-seq Data Analysis

Since methylated cell-free nucleic acids were sequenced, the obtained nucleic acid fragment data are in a methylated state, and can be aligned to the human reference genome, thereby identifying methylated regions in the entire human genome. In EM-Seq data, methylated cytosine remains as cytosine, and unmethylated cytosine is converted to thymine, so that methylated CpGs and the degree of methylation can be identified. Differentially methylated CpGs between the lung cancer group and the normal group were selected.

EM-seq data were divided into discovery and external groups as shown in Tables 7 and 8 below. Marker selection was performed using the whole genome EM-seq data, feature filtering was performed using the external group of the twist methylome panel, and the presence or absence of cancer was classified using the entire twist methylome panel data.

[Table 7]

| WGEM-seq | Lung cancer tissue | Normal tissue | WBC | Total |
|---|---|---|---|---|
| Total | 7 | 7 | 10 | 24 |

[Table 8]

| Panel | Lung cancer | Normal | Total |
|---|---|---|---|
| Discovery | 7 | - | 7 |
| External | 5 | 7 | 12 |
| Total | 12 | 7 | 19 |

First, adapter trimming and quality trimming of fastq files were performed using Trim Galore (version 0.6.6), and then nucleic acid fragment data were aligned to the human reference genome (hg19) using the Bismark (version 0.23.0) alignment tool. Only nucleic acid fragments with a mapping quality of 10 or higher were selected using the Samtools view (version 1.11) tool, and chr1-22, X, Y were retained. Then, methylation calling was performed using bismark_methylation_extractor of Bismark (version 0.23.0).

The beta values (methylation percentage) of the tumor and normal samples were merged into a single file using the methylKit (version 1.12.0) R package, and then CpGs with an absolute value of difference exceeding 25 and a qvalue of less than 0.01 were selected using the methylKit (version 1.12.0) R package.

As a result of selecting differentially methylated CpGs between lung tissue and surrounding normal tissue using the above method, 72,113 hypermethylated CpGs and 260,606 hypomethylated CpGs specific to lung cancer were selected, and as a result of selecting differentially methylated CpGs between lung normal tissue and normal whole blood (WBC), 108,548 hypermethylated CpGs and 387,282 hypomethylated CpGs specific to lung tissue were selected.

Among the above-described selected CpGs, overlapping CpGs were selected, thereby selecting 13,196 hypermethylated CpGs and 54,239 hypomethylated CpGs.

### Example 6. Final Marker Selection

138 hypermethylated CpGs and 1,309 hypomethylated CpGs found identically in Examples 1 and 3 were selected, and 362 hypermethylated CpGs and 101 hypomethylated CpGs found identically in Examples 1 and 5 were selected, and then integrated without duplication, thereby selecting 489 hypermethylated CpGs and 1,401 hypomethylated CpGs as primary comprehensive regions.

To more specifically select lung cancer-specific CpGs among the selected primary comprehensive CpGs, a filtering process based on the external set of EM-Seq data using the cfDNA Targeted methylome panel in Table 6 above was performed.

First, the AUC that distinguishes the lung cancer group from the normal group was calculated using the beta value of the primary comprehensive CpGs, and significant CpGs with an AUC of 6.5 or higher were selected.

In addition, CpGs were selected in which the absolute value of the difference between the lung cancer group and the normal group was 3 or more, the absolute value of the q-value was less than 0.05, and the absolute value of the standard deviation (SD) of the normal group was less than 0.05.

The CpGs selected in the two filtering steps were combined without duplication, thereby finally selecting 130 hypermethylated CpGs and 236 hypomethylated CpGs.

The list of 366 selected methylation markers is shown in Table 9 below.

[Table 9]

| Chromosome | Start position | End position | Gene | illumina ProbID | gain/loss |
|---|---|---|---|---|---|
| chr1 | 38412711 | 38412712 | INPP5B | cg17949727,cg10784030 | gain |
| chr1 | 63790044 | 63790045 | FOXD3 | . | gain |
| chr1 | 75595970 | 75595971 | LHX8 | . | gain |
| chr1 | 79472282 | 79472283 | ADGRL4 | cg14319235 | gain |
| chr1 | 79472343 | 79472344 | ADGRL4 | cg21113740,cg04360793 | gain |
| chr1 | 79472361 | 79472362 | ADGRL4 | cg04360793,cg15084543 | gain |
| chr1 | 79472408 | 79472409 | ADGRL4 | cg15084543 | gain |
| chr1 | 79472452 | 79472453 | ADGRL4 | . | gain |
| chr1 | 110611218 | 110611219 | ALX3 | cg11071207 | gain |
| chr1 | 119526060 | 119526061 | TBX15 | . | gain |
| chr1 | 119529219 | 119529220 | TBX15 | cg05363616 | gain |
| chr1 | 119532093 | 119532094 | TBX15 | cg24884142 | gain |
| chr1 | 119535928 | 119535929 | TBX15 | . | gain |
| chr1 | 119548825 | 119548826 | . | cg01959730 | gain |
| chr1 | 156357839 | 156357840 | . | cg22353551,cg17966560 | gain |
| chr1 | 157164796 | 157164797 | . | cg09504320 | gain |
| chr1 | 170630070 | 170630071 | PRRX1 | . | gain |
| chr1 | 214153294 | 214153295 | . | . | gain |
| chr1 | 214153460 | 214153461 | . | . | gain |
| chr1 | 214153472 | 214153473 | . | cg25742246 | gain |
| chr10 | 81003175 | 81003176 | ZMIZ1 | cg14371731 | gain |
| chr10 | 103043991 | 103043992 | . | cg01812599,cg19507206 | gain |
| chr10 | 118892211 | 118892212 | VAX1 | . | gain |
| chr11 | 31819444 | 31819445 | PAX6 | cg21016855,cg06666008 | gain |
| chr12 | 54339653 | 54339654 | HOXC13 | cg03239552 | gain |
| chr12 | 85667616 | 85667617 | . | cg16733705 | gain |
| chr12 | 85671811 | 85671812 | ALX1 | . | gain |
| chr12 | 85673221 | 85673222 | ALX1 | . | gain |
| chr13 | 79169872 | 79169873 | OBI1-AS1 | . | gain |
| chr13 | 112726118 | 112726119 | . | cg14582400 | gain |
| chr13 | 112759719 | 112759720 | . | cg13692446 | gain |

(continued)

| Chromosome | Start position | End position | Gene | illumina ProbID | gain/loss |
|---|---|---|---|---|---|
| chr14 | 29235193 | 29235194 | FOXG1 | . | gain |
| chr14 | 29235196 | 29235197 | FOXG1 | cg25078444 | gain |
| chr14 | 29254942 | 29254943 | LINC01551 | cg11226913 | gain |
| chr14 | 57265875 | 57265876 | . | cg18280830,cg27175093 | gain |
| chr14 | 57278188 | 57278189 | OTX2-AS1 | cg18451814 | gain |
| chr14 | 99729088 | 99729089 | BCL11B | . | gain |
| chr14 | 101923256 | 101923257 | . | . | gain |
| chr16 | 66613266 | 66613267 | CMTM2 | cg16626067,cg09854734, cg06666025 | gain |
| chr16 | 86547530 | 86547531 | FOXF 1 | cg00551679,cg02783918 | gain |
| chr19 | 11785188 | 11785189 | ZNF833P | . | gain |
| chr19 | 12624466 | 12624467 | ZNF709 | . | gain |
| chr19 | 22806184 | 22806185 | . | . | gain |
| chr19 | 58220494 | 58220495 | ZNF776 | . | gain |
| chr19 | 58220516 | 58220517 | ZNF776 | cg21790626 | gain |
| chr19 | 58220657 | 58220658 | ZNF776 | . | gain |
| chr19 | 58220662 | 58220663 | ZNF776 | cg03234186 | gain |
| chr19 | 58220773 | 58220774 | ZNF776 | . | gain |
| chr2 | 45159894 | 45159895 | . | . | gain |
| chr2 | 63281844 | 63281845 | OTX1 | cg00445405 | gain |
| chr2 | 66666684 | 66666685 | MEISI | . | gain |
| chr2 | 73148229 | 73148230 | EMX1 | . | gain |
| chr2 | 74875227 | 74875228 | MIAP | cg20945085 | gain |
| chr2 | 119599459 | 119599460 | . | cg20175390 | gain |
| chr2 | 119607379 | 119607380 | EN1 | cg03726556,cg14228146 | gain |
| chr2 | 119607573 | 119607574 | EN1 | cg117030233,cg18093372 | gain |
| chr2 | 171676809 | 171676810 | GAD1 | . | gain |
| chr2 | 172952948 | 172952949 | DLX1 | . | gain |
| chr2 | 176948728 | 176948729 | EVX2 | cg01749491,cg11359133, cg07411620 | gain |
| chr2 | 176981336 | 176981337 | HOXD10 | cg05979020 | gain |
| chr2 | 176986460 | 176986461 | HOXD9 | cg08363794 | gain |
| chr2 | 176993017 | 176993018 | HOXD8 | . | gain |
| chr2 | 177012191 | 177012192 | MIR10B | cg04194947 | gain |
| chr2 | 177014996 | 177014997 | MIR10B | . | gain |
| chr2 | 177015044 | 177015045 | MIR10B | cg25942990 | gain |
| chr2 | 177027043 | 177027044 | HOXD3 | cg06316886 | gain |
| chr2 | 177029073 | 177029074 | HOXD3 | . | gain |
| chr2 | 177029459 | 177029460 | HOXD3 | cg24541426 | gain |

(continued)

| Chromosome | Start position | End position | Gene | illumina ProbID | gain/loss |
|---|---|---|---|---|---|
| chr2 | 177029608 | 177029609 | HOXD3 | . | gain |
| chr2 | 182543233 | 182543234 | NEUROD1 | cg16640855 | gain |
| chr20 | 43726765 | 43726766 | K CNS 1 | cg07160746 | gain |
| chr3 | 138662315 | 138662316 | FOXL2NB | cg15931949 | gain |
| chr3 | 147111660 | 147111661 | ZIC4 | . | gain |
| chr3 | 147113918 | 147113919 | ZIC4 | cg12892506 | gain |
| chr3 | 147114406 | 147114407 | ZIC4 | cg00028935 | gain |
| chr3 | 147128157 | 147128158 | ZIC4 | cg12595013 | gain |
| chr3 | 147130536 | 147130537 | ZIC1 | cg19029181 | gain |
| chr3 | 147142182 | 147142183 | . | cg01150433 | gain |
| chr3 | 157812226 | 157812227 | . | . | gain |
| chr3 | 157813327 | 157813328 | . | . | gain |
| chr3 | 157820591 | 157820592 | SHOX2 | cg18194945 | gain |
| chr3 | 157821262 | 157821263 | SHOX2 | cg04521004 | gain |
| chr3 | 157821994 | 157821995 | SHOX2 | cg04913979 | gain |
| chr3 | 181442676 | 181442677 | SOX2-OT | cg01783662,cg11362010 | gain |
| chr4 | 11370466 | 11370467 | MIR572 | cg11636702 | gain |
| chr4 | 15704680 | 15704681 | BST1 | cg00731785 | gain |
| chr4 | 41875470 | 41875471 | . | cg05036656 | gain |
| chr4 | 85402870 | 85402871 | . | . | gain |
| chr4 | 111543401 | 111543402 | PITX2 | cg05835105 | gain |
| chr4 | 111552040 | 111552041 | PITX2 | cg26708319 | gain |
| chr4 | 174452835 | 174452836 | HAND2-AS1 | . | gain |
| chr5 | 42952113 | 42952114 | . | . | gain |
| chr5 | 54519023 | 54519024 | MCIDAS | cg04286194 | gain |
| chr5 | 76932016 | 76932017 | OTP | . | gain |
| chr5 | 76934327 | 76934328 | OTP | . | gain |
| chr5 | 92909429 | 92909430 | NR2F1-AS1 | cg15143788 | gain |
| chr5 | 140536238 | 140536239 | PCDHB 17P | . | gain |
| chr5 | 172671754 | 172671755 | . | . | gain |
| chr5 | 172672766 | 172672767 | . | . | gain |
| chr6 | 10425648 | 10425649 | . | cg22367191 | gain |
| chr6 | 27648004 | 27648005 | . | . | gain |
| chr6 | 50804147 | 50804148 | TFAP2B | cg24641186 | gain |
| chr6 | 100050791 | 100050792 | PRDM13 | cg27112666,cg04670802 | gain |
| chr6 | 100066698 | 100066699 | . | cg00792513 | gain |
| chr6 | 100903817 | 100903818 | SIM1 | . | gain |
| chr6 | 100917397 | 100917398 | SIM1 | . | gain |
| chr6 | 105388731 | 105388732 | LIN28B-AS1 | cg03738025 | gain |

(continued)

| Chromosome | Start position | End position | Gene | illumina ProbID | gain/loss |
|---|---|---|---|---|---|
| chr6 | 134210946 | 134210947 | TCF21 | . | gain |
| chr7 | 8482235 | 8482236 | NXPH1 | cg12597389 | gain |
| chr7 | 8482325 | 8482326 | NXPH1 | . | gain |
| chr7 | 25898688 | 25898689 | . | cg03190219 | gain |
| chr7 | 26897202 | 26897203 | SKAP2 | cg09426383 | gain |
| chr7 | 26897253 | 26897254 | SKAP2 | cg20747577 | gain |
| chr7 | 27192056 | 27192057 | HOXA-AS3 | cg10739556,cg24398479 | gain |
| chr7 | 27205217 | 27205218 | HOXA9 | cg05065989 | gain |
| chr7 | 27206073 | 27206074 | HOXA9 | cg15506609 | gain |
| chr7 | 27225523 | 27225524 | HOXA 11-AS | cg10657141 | gain |
| chr7 | 27225528 | 27225529 | HOXA 11-AS | cg10657141,cg05311410 | gain |
| chr7 | 27225543 | 27225544 | HOXA 11-AS | cg10657141,cg05311410, cg17466857 | gain |
| chr7 | 27225772 | 27225773 | HOXA 11-AS | cg07116997 | gain |
| chr7 | 39649443 | 39649444 | LOC646999 | . | gain |
| chr7 | 96631680 | 96631681 | DLX6-AS1 | . | gain |
| chr7 | 96636616 | 96636617 | DLX6-AS1 | cg03774463 | gain |
| chr7 | 96642794 | 96642795 | DLX6-AS1 | cg10205928 | gain |
| chr7 | 129423053 | 129423054 | . | . | gain |
| chr7 | 153584582 | 153584583 | DPP6 | cg06495961,cg14523847, cg27032232 | gain |
| chr7 | 153584597 | 153584598 | DPP6 | cg14523847,cg27032232 | gain |
| chr8 | 4849522 | 4849523 | CSMD1 | . | gain |
| chr8 | 65498812 | 65498813 | LOC401463 | . | gain |
| chr9 | 79638117 | 79638118 | . | cg00959431 | gain |
| chr1 | 942161 | 942162 | . | cg13219080,cg09364122 | loss |
| chr1 | 1267193 | 1267194 | TAS1R3 | cg13246026 | loss |
| chr1 | 1267201 | 1267202 | TAS1R3 | . | loss |
| chr1 | 2345333 | 2345334 | PEX10 | . | loss |
| chr1 | 2750406 | 2750407 | . | cg06711333 | loss |
| chr1 | 2764669 | 2764670 | . | . | loss |
| chr1 | 2847632 | 2847633 | . | cg14165772 | loss |
| chr1 | 2850512 | 2850513 | . | . | loss |
| chr1 | 2904595 | 2904596 | . | . | loss |
| chr1 | 2936277 | 2936278 | ACTRT2 | cg17025560,cg14093144 | loss |
| chr1 | 2949633 | 2949634 | . | cg06201717,cg19825898, cg24150662 | loss |
| chr1 | 3058151 | 3058152 | PRDM16 | cg05343184 | loss |
| chr1 | 3083061 | 3083062 | PRDM16 | cg03391389 | loss |

(continued)

| Chromosome | Start position | End position | Gene | illumina ProbID | gain/loss |
|---|---|---|---|---|---|
| chr1 | 3150935 | 3150936 | PRDM16 | . | loss |
| chr1 | 3210325 | 3210326 | PRDM16 | cg10328768 | loss |
| chr1 | 3495611 | 3495612 | MEGF6 | . | loss |
| chr1 | 3606892 | 3606893 | TP73 | cg23163013 | loss |
| chr1 | 9896309 | 9896310 | . | cg20488341 | loss |
| chr1 | 22925489 | 22925490 | EPHA8 | cg22493620,cg09940915 | loss |
| chr1 | 35220814 | 35220815 | GJB4 | cg01333788,cg27632959 | loss |
| chr1 | 56880606 | 56880607 | . | cg18090390 | loss |
| chr10 | 44197967 | 44197968 | . | cg02112154 | loss |
| chr10 | 134652276 | 134652277 | CFAP46 | . | loss |
| chr10 | 134833372 | 134833373 | . | cg18720533,cg02401524 | loss |
| chr10 | 134833874 | 134833875 | . | cg03030984 | loss |
| chr10 | 134838656 | 134838657 | . | . | loss |
| chr10 | 134842688 | 134842689 | . | . | loss |
| chr10 | 134912012 | 134912013 | ADGRA1 | . | loss |
| chr10 | 134928457 | 134928458 | ADGRA1 | . | loss |
| chr10 | 134929181 | 134929182 | ADGRA1 | . | loss |
| chr10 | 135015300 | 135015301 | KNDC1 | . | loss |
| chr10 | 135016451 | 135016452 | KNDC1 | . | loss |
| chr10 | 135018765 | 135018766 | KNDC1 | . | loss |
| chr10 | 135018844 | 135018845 | KNDC1 | . | loss |
| chr11 | 393606 | 393607 | PKP3 | cg03547062,cg18308516 | loss |
| chr11 | 1006121 | 1006122 | AP2A2 | cg24716664 | loss |
| chr11 | 1050848 | 1050849 | . | . | loss |
| chr11 | 1257516 | 1257517 | MUC5B | . | loss |
| chr11 | 1464300 | 1464301 | BRSK2 | . | loss |
| chr11 | 2033843 | 2033844 | . | cg25068071 | loss |
| chr11 | 2182540 | 2182541 | INS | . | loss |
| chr11 | 47359115 | 47359116 | MYBPC3 | cg05649391 | loss |
| chr11 | 61302186 | 61302187 | SYT7 | . | loss |
| chr11 | 66104485 | 66104486 | RIN1 | cg14042308,cg23220439 | loss |
| chr11 | 68964113 | 68964114 | . | . | loss |
| chr11 | 69192623 | 69192624 | . | . | loss |
| chr11 | 69192697 | 69192698 | . | cg03888946 | loss |
| chr11 | 70781118 | 70781119 | SHANK2 | . | loss |
| chr11 | 120008998 | 120008999 | TRIM29 | cg24593464,cg02280 132 | loss |
| chr11 | 128812875 | 128812876 | TP53AIP1 | cg04817300,cg22837015, cg19275133 | loss |
| chr12 | 1943889 | 1943890 | CACNA2D4 | cg02505216 | loss |

(continued)

| Chromosome | Start position | End position | Gene | illumina ProbID | gain/loss |
|---|---|---|---|---|---|
| chr12 | 2613914 | 2613915 | CACNA1C | cg02140020,cg12205822 | loss |
| chr12 | 29650795 | 29650796 | OVCH1 | cg07854132,cg22535349 | loss |
| chr12 | 94954828 | 94954829 | MIR5700 | . | loss |
| chr12 | 124693173 | 124693174 | ZNF664-RFLNA | cg05619449 | loss |
| chr12 | 131580615 | 131580616 | ADGRD1 | cg24098252 | loss |
| chr12 | 132680972 | 132680973 | GALNT9 | cg03225476 | loss |
| chr12 | 132839436 | 132839437 | GALNT9 | . | loss |
| chr12 | 132847907 | 132847908 | GALNT9 | . | loss |
| chr12 | 132892613 | 132892614 | GALNT9 | cg08899593 | loss |
| chr12 | 133028690 | 133028691 | . | . | loss |
| chr13 | 109741117 | 109741118 | MYO16 | . | loss |
| chr13 | 109741176 | 109741177 | MYO16 | . | loss |
| chr13 | 112200864 | 112200865 | . | . | loss |
| chr13 | 112607846 | 112607847 | . | cg08644897 | loss |
| chr13 | 114056351 | 114056352 | . | . | loss |
| chr13 | 114065008 | 114065009 | . | . | loss |
| chr13 | 114065669 | 114065670 | . | . | loss |
| chr13 | 114311898 | 114311899 | ATP4B | cg17363387 | loss |
| chr14 | 77328166 | 77328167 | LRRC74A | cg18269382 | loss |
| chr14 | 100658902 | 100658903 | . | cg23112821,cg01538305 | loss |
| chr14 | 101334582 | 101334583 | MIR493 | . | loss |
| chr14 | 104648347 | 104648348 | . | . | loss |
| chr14 | 104837541 | 104837542 | . | cg08037115 | loss |
| chr14 | 104837550 | 104837551 | . | . | loss |
| chr14 | 105031243 | 105031244 | . | cg10426658 | loss |
| chr14 | 106174668 | 106174669 | . | cg16279237 | loss |
| chr14 | 106239286 | 106239287 | . | . | loss |
| chr14 | 106320669 | 106320670 | . | cg23684139 | loss |
| chr15 | 26235162 | 26235163 | LINC02346 | . | loss |
| chr15 | 78505051 | 78505052 | ACSBG1 | . | loss |
| chr15 | 89386800 | 89386801 | ACAN | . | loss |
| chr16 | 605214 | 605215 | . | cg10396171 | loss |
| chr16 | 605347 | 605348 | . | . | loss |
| chr16 | 644081 | 644082 | RAB40C | . | loss |
| chr16 | 1052939 | 1052940 | . | cg08438529 | loss |
| chr16 | 1061053 | 1061054 | . | cg05970874 | loss |
| chr16 | 1093823 | 1093824 | . | cg10129154 | loss |
| chr16 | 1147829 | 1147830 | C1QTNF8 | cg27136719 | loss |

(continued)

| Chromosome | Start position | End position | Gene | illumina ProbID | gain/loss |
|---|---|---|---|---|---|
| chr16 | 1233872 | 1233873 | CACNA1H | cg06008356 | loss |
| chr16 | 1256295 | 1256296 | CACNA1H | cg09842735 | loss |
| chr16 | 1257884 | 1257885 | CACNA1H | cg16453617 | loss |
| chr16 | 1271741 | 1271742 | CACNA1H | . | loss |
| chr16 | 1271998 | 1271999 | TPSG1 | cg05042366 | loss |
| chr16 | 1316081 | 1316082 | . | cg08197072 | loss |
| chr16 | 2213440 | 2213441 | TRAF7 | cg04987122 | loss |
| chr16 | 2213749 | 2213750 | TRAF7 | cg02925248 | loss |
| chr16 | 11770950 | 11770951 | SNN | . | loss |
| chr16 | 22326535 | 22326536 | POLR3E | cg10508883 | loss |
| chr16 | 81564192 | 81564193 | CMIP | cg01033642 | loss |
| chr16 | 85019773 | 85019774 | ZDHHC7 | . | loss |
| chr16 | 85678884 | 85678885 | GSE1 | cg01767053 | loss |
| chr16 | 86465884 | 86465885 | . | cg01882058 | loss |
| chr16 | 87682572 | 87682573 | JPH3 | cg05443795 | loss |
| chr16 | 87958407 | 87958408 | CA5A | cg04352288 | loss |
| chr16 | 87958493 | 87958494 | CA5A | cg06948222 | loss |
| chr16 | 88442086 | 88442087 | . | . | loss |
| chr16 | 88460610 | 88460611 | . | . | loss |
| chr16 | 89016957 | 89016958 | CBFA2T3 | . | loss |
| chr16 | 89029055 | 89029056 | CBFA2T3 | cg06982885 | loss |
| chr16 | 89110004 | 89110005 | . | . | loss |
| chr16 | 89118838 | 89118839 | . | . | loss |
| chr16 | 89245231 | 89245232 | CDH15 | . | loss |
| chr16 | 89704682 | 89704683 | DPEP1 | . | loss |
| chr17 | 38860042 | 38860043 | KRT24 | cg04219544 | loss |
| chr17 | 40705588 | 40705589 | HSD17B1 | cg01612247 | loss |
| chr17 | 48680554 | 48680555 | CACNA1G | eg26639906 | loss |
| chr17 | 49027095 | 49027096 | . | eg00029979 | loss |
| chr17 | 77174120 | 77174121 | RBFOX3 | . | loss |
| chr17 | 79096529 | 79096530 | AATK | . | loss |
| chr17 | 79099882 | 79099883 | AATK | . | loss |
| chr17 | 79652244 | 79652245 | ARL 16 | cg22209573 | loss |
| chr17 | 79961522 | 79961523 | ASPSCR1 | cg00598204 | loss |
| chr17 | 80342459 | 80342460 | . | eg05995260 | loss |
| chr18 | 44259973 | 44259974 | ST8SIA5 | . | loss |
| chr18 | 61817320 | 61817321 | LINC00305 | cg18827787 | loss |
| chr18 | 76485587 | 76485588 | . | eg22110922 | loss |
| chr19 | 3936724 | 3936725 | NMRK2 | . | loss |

(continued)

| Chromosome | Start position | End position | Gene | illumina ProbID | gain/loss |
|---|---|---|---|---|---|
| chr19 | 4552496 | 4552497 | SEMA6B | . | loss |
| chr19 | 13394116 | 13394117 | CACNA1A | cg16707895 | loss |
| chr19 | 18085190 | 18085191 | KCNN1 | eg27648056 | loss |
| chr19 | 57175043 | 57175044 | ZNF835 | cg08431567,cg06230408 | loss |
| chr2 | 1133947 | 1133948 | SNTG2 | cg10325383 | loss |
| chr2 | 1880012 | 1880013 | MYT1L | eg22946562 | loss |
| chr2 | 4931004 | 4931005 | . | . | loss |
| chr2 | 4931074 | 4931075 | . | cg02883150 | loss |
| chr2 | 10638113 | 10638114 | . | cg22157027 | loss |
| chr2 | 10666713 | 10666714 | . | cg08846959 | loss |
| chr2 | 26800399 | 26800400 | FAM166C | . | loss |
| chr2 | 233198590 | 233198591 | DIS3L2 | . | loss |
| chr2 | 239544973 | 239544974 | . | . | loss |
| chr2 | 241083864 | 241083865 | OTOS | . | loss |
| chr20 | 5451900 | 5451901 | LOC643406 | cg04281261 | loss |
| chr20 | 60120472 | 60120473 | CDH4 | cg12762862 | loss |
| chr20 | 60456498 | 60456499 | CDH4 | cg24688309,cg01185502 | loss |
| chr20 | 60456503 | 60456504 | CDH4 | cg01185502 | loss |
| chr20 | 61625364 | 61625365 | . | cg10233904 | loss |
| chr20 | 61695598 | 61695599 | LINC01749 | cg01042841 | loss |
| chr20 | 61755045 | 61755046 | . | cg16473797 | loss |
| chr20 | 61792443 | 61792444 | . | . | loss |
| chr20 | 62079911 | 62079912 | KCNQ2 | . | loss |
| chr21 | 46420451 | 46420452 | PICSAR | . | loss |
| chr21 | 46677879 | 46677880 | . | cg10272901 | loss |
| chr22 | 29704483 | 29704484 | GAS2L1 | eg04929173 | loss |
| chr22 | 37769171 | 37769172 | ELFN2 | cg21515305 | loss |
| chr22 | 38506737 | 38506738 | BAIAP2L2 | cg08196512,cg11744966, cg09247692 | loss |
| chr22 | 43835600 | 43835601 | MPPED1 | . | loss |
| chr22 | 44708861 | 44708862 | SHISAL 1 | . | loss |
| chr22 | 49137906 | 49137907 | TAFA5 | cg12986453 | loss |
| chr22 | 49376886 | 49376887 | . | . | loss |
| chr22 | 49487343 | 49487344 | . | . | loss |
| chr22 | 50470101 | 50470102 | . | . | loss |
| chr3 | 14615134 | 14615135 | . | cg11100465 | loss |
| chr3 | 128182392 | 128182393 | DNAJB8 | cg20268341 | loss |
| chr3 | 134146405 | 134146406 | . | . | loss |
| chr3 | 194078147 | 194078148 | LRRC15 | cg19531604 | loss |

(continued)

| Chromosome | Start position | End position | Gene | illumina ProbID | gain/loss |
|---|---|---|---|---|---|
| chr3 | 196065306 | 196065307 | TM4SF19 | cg21845080 | loss |
| chr3 | 196065318 | 196065319 | TM4SF19 | . | loss |
| chr3 | 196065320 | 196065321 | TM4SF19 | cg22496559 | loss |
| chr3 | 196065328 | 196065329 | TM4SF19 | cg22496559,cg11363229 | loss |
| chr3 | 196065569 | 196065570 | TM4SF19 | . | loss |
| chr4 | 1538525 | 1538526 | . | . | loss |
| chr5 | 476497 | 476498 | PP7080 | eg06852942 | loss |
| chr5 | 476646 | 476647 | PP7080 | . | loss |
| chr5 | 555251 | 555252 | . | . | loss |
| chr5 | 1003897 | 1003898 | . | cg18801579 | loss |
| chr5 | 1119025 | 1119026 | . | . | loss |
| chr5 | 1140096 | 1140097 | . | cg01056615,cg23499632 | loss |
| chr5 | 1202449 | 1202450 | SLC6A19 | cg24041118 | loss |
| chr5 | 1219972 | 1219973 | SLC6A19 | cg24114651 | loss |
| chr5 | 1232590 | 1232591 | SLC6A18 | cg11231434 | loss |
| chr5 | 1244729 | 1244730 | SLC6A18 | cg06708198 | loss |
| chr5 | 1257084 | 1257085 | TERT | cg17249224 | loss |
| chr5 | 1767994 | 1767995 | . | cg10452667 | loss |
| chr5 | 2103257 | 2103258 | . | cg19021412 | loss |
| chr5 | 2132336 | 2132337 | . | . | loss |
| chr5 | 17654594 | 17654595 | . | cg19090585 | loss |
| chr5 | 169531595 | 169531596 | FOXII | . | loss |
| chr5 | 180046901 | 180046902 | FLT4 | cg23297812 | loss |
| chr5 | 180047184 | 180047185 | FLT4 | cg07072463 | loss |
| chr6 | 466893 | 466894 | . | . | loss |
| chr6 | 25218752 | 25218753 | . | . | loss |
| chr6 | 169740781 | 169740782 | . | cg11583041 | loss |
| chr7 | 1303351 | 1303352 | . | cg23167506 | loss |
| chr7 | 1403804 | 1403805 | . | . | loss |
| chr7 | 4841835 | 4841836 | RADIL | . | loss |
| chr7 | 71177239 | 71177240 | GALNT17 | cg08766170 | loss |
| chr7 | 154542060 | 154542061 | DPP6 | cg08910550 | loss |
| chr7 | 155191845 | 155191846 | . | . | loss |
| chr7 | 157085979 | 157085980 | . | . | loss |
| chr7 | 157502455 | 157502456 | PTPRN2 | cg13401196 | loss |
| chr7 | 157544912 | 157544913 | PTPRN2 | cg22377978 | loss |
| chr7 | 157776887 | 157776888 | PTPRN2 | cg23684218 | loss |
| chr7 | 157968578 | 157968579 | PTPRN2 | . | loss |
| chr7 | 158030726 | 158030727 | PTPRN2 | . | loss |

(continued)

| Chromosome | Start position | End position | Gene | illumina ProbID | gain/loss |
|---|---|---|---|---|---|
| chr7 | 158331217 | 158331218 | PTPRN2 | cg12364136 | loss |
| chr7 | 158799715 | 158799716 | LINC00689 | cg11772799,cg10874111 | loss |
| chr7 | 158820591 | 158820592 | LINC00689 | . | loss |
| chr8 | 978226 | 978227 | . | cg04544946 | loss |
| chr8 | 1140682 | 1140683 | . | cg20860124 | loss |
| chr8 | 10753237 | 10753238 | . | . | loss |
| chr8 | 141107113 | 141107114 | TRAPPC9 | . | loss |
| chr8 | 142517494 | 142517495 | MROH5 | cg26654519 | loss |
| chr8 | 142841913 | 142841914 | . | cg07969668 | loss |
| chr8 | 142841965 | 142841966 | . | . | loss |
| chr8 | 143201145 | 143201146 | . | . | loss |
| chr8 | 143208101 | 143208102 | . | cg00584485 | loss |
| chr8 | 143262200 | 143262201 | . | eg03957095 | loss |
| chr8 | 143333654 | 143333655 | TSNARE1 | cg08957001 | loss |
| chr8 | 143509263 | 143509264 | . | . | loss |
| chr8 | 143535735 | 143535736 | . | . | loss |
| chr8 | 143605535 | 143605536 | ADGRB1 | . | loss |
| chr8 | 143625791 | 143625792 | ADGRB1 | eg27039312 | loss |
| chr8 | 143915919 | 143915920 | GML | cg07711097 | loss |
| chr8 | 143915954 | 143915955 | GML | cg07711097,cg22396979 | loss |
| chr8 | 143961145 | 143961146 | CYP11B1 | . | loss |
| chr8 | 144203483 | 144203484 | . | . | loss |
| chr8 | 144366016 | 144366017 | . | . | loss |
| chr8 | 144416327 | 144416328 | TOP1MT | . | loss |
| chr8 | 144801223 | 144801224 | MAPK15 | cg06344474 | loss |
| chr8 | 145697495 | 145697496 | KIFC2 | eg06666727 | loss |
| chr8 | 145758486 | 145758487 | ARHGAP39 | cg25667997 | loss |
| chr9 | 137718814 | 137718815 | COL5A1 | . | loss |
| chr9 | 138018566 | 138018567 | . | cg13982366 | loss |
| chr9 | 140041732 | 140041733 | GRIN1 | . | loss |
| chr9 | 140333139 | 140333140 | ENTPD8 | . | loss |

## Example 7. Evaluation of Marker Performance

The performance of the 366 markers selected in Example 6 was evaluated using the beta value obtained from the entire EM-Seq data. First, the standard deviation and mean of the normal group samples were calculated, and then the z-score for each marker was calculated using Equation 1 below.

Equation 1:

$$Z\,score = \frac{\text{methylation level in step (b)} - \text{mean of methylation levels in normal samples}}{\text{standard deviation of methylation levels in normal samples}}$$

Using the calculated z-scores, the mean, sum, and SD of z-scores were calculated, and if the calculated mean, sum, and SD of z-scores exceeded the cut-off values shown in Table 10 below, the presence of lung cancer was determined.

Each of the cut-off value in Table 10 was determined as the 95 percentile value of normal samples.

[Table 10]

| Score | Cut-off |
|---|---|
| **Mean of Z-score** | 0.53 |
| **Sum of Z-score** | 195.04 |
| **SD of Z-score** | 1.06 |

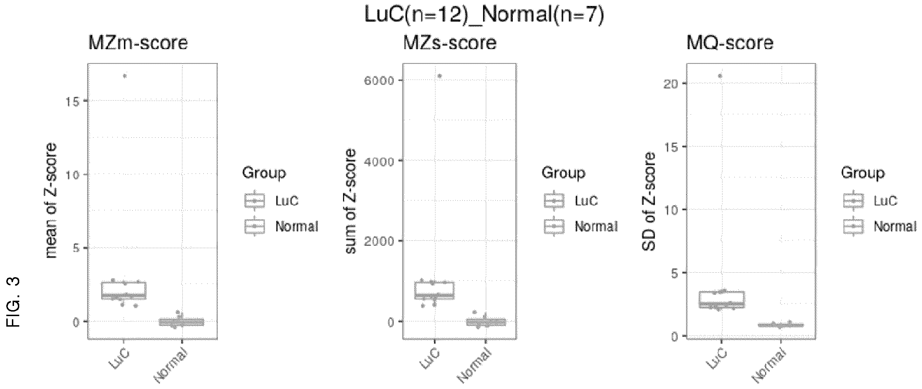

As a result, as shown in Table 11 below and FIGS. 2 and 3, it was confirmed that the AUC values, which are the results of ROC analysis, were 1.00, 1.00 and 1.00 for the mean of Z-scores, the sum of Z-scores and the SD of Z-scores, respectively.

[Table 11]

| Score | AUC |
|---|---|
| **Mean of Z-scores** | 1.00 |
| **Sum of Z-scores** | 1.00 |
| **SD of Z-scores** | 1.00 |

## Example 8. Selection of Lung Cancer-Specific Methylation Regions from TCGA Methylation 450K Array Data

In addition, to obtain a minimal marker combination for diagnosing lung cancer, the degree of methylation was measured using the Infinium Human Methylation 450K BeadChip array data (UCSC Xena, http://xena.ucsc.edu) from The Cancer Genome Atlas (TCGA). DNA extracted from tissue is converted by bisulfite treatment, and DNA methylation can be determined through modification of cytosine bases. The degree of methylation can be determined for each region, and the beta value, which represents the degree of methylation, was used to select differentially methylated regions between lung cancer tissue and surrounding normal tissue.

TCGA methylation 450k array data are shown in Table 12 below.

[Table 12]

| | **Primary Solid Tumor** | **Solid Tissue Normal** | **Total** |
|---|---|---|---|
| LUAD | 458 | 32 | 490 |
| LUSC | 370 | 42 | 412 |
| **Total** | 828 | 74 | 902 |

First, missing values were excluded from about 480k sites, and then the methylation score was used and calculated to select significantly hypermethylated regions. The methylation score of hypermethylated regions was calculated by adding the beta value, and the methylation score of hypomethylated regions was calculated by multiplying the beta value by -1 and then calculating the sum.

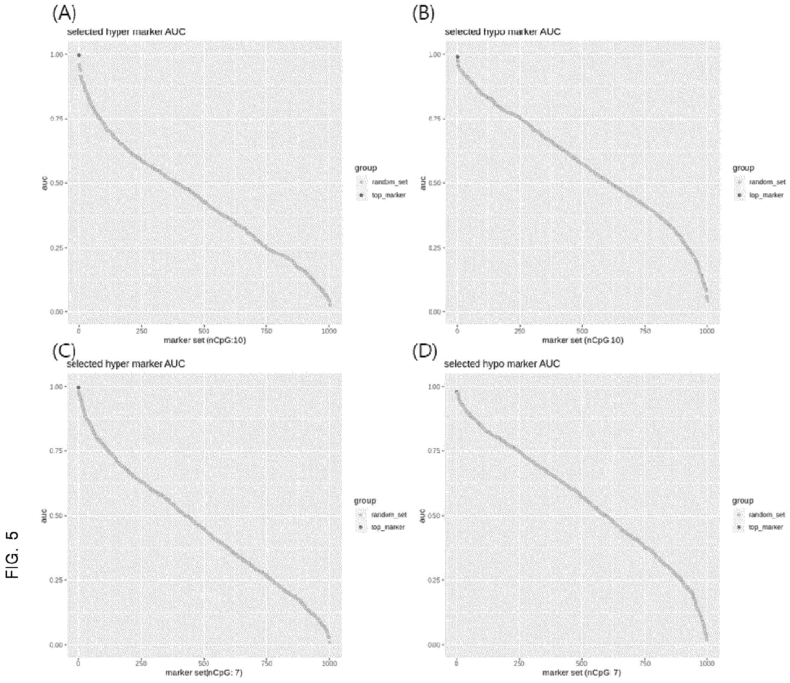

Sets of CpG sites were constructed by randomly sampling 10 CpG sites 1000 times with replacement, and then each set of CpG sites was used to calculate the methylation scores of 902 lung cancer tissues and surrounding normal tissues. The calculated methylation scores were used to calculate the AUC for distinguishing between lung cancer tissues and surrounding normal tissues, and the CpG site with the highest AUC was selected. As a result, a set of 10 hypermethylated regions with an AUC of 0.996 and a set of 10 hypomethylated regions with an AUC of 0.990 were selected (FIGS. 5A and 5B) .

In addition, sets of CpG sites were constructed by randomly sampling 7 CpG sites 1,000 times with replacement, and then a set of 7 hypermethylated regions with an AUC of 0.996 and a set of 7 hypomethylated regions with an AUC of 0.976 were selected using the same method (FIGS. 5C and 5D and Table 13).

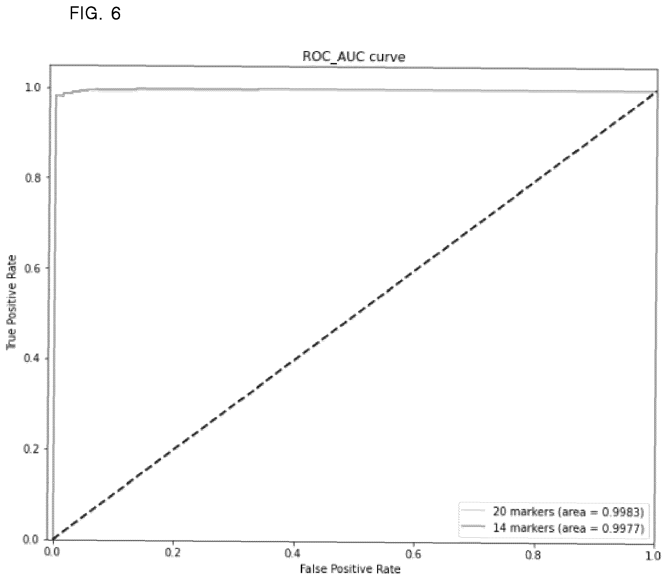

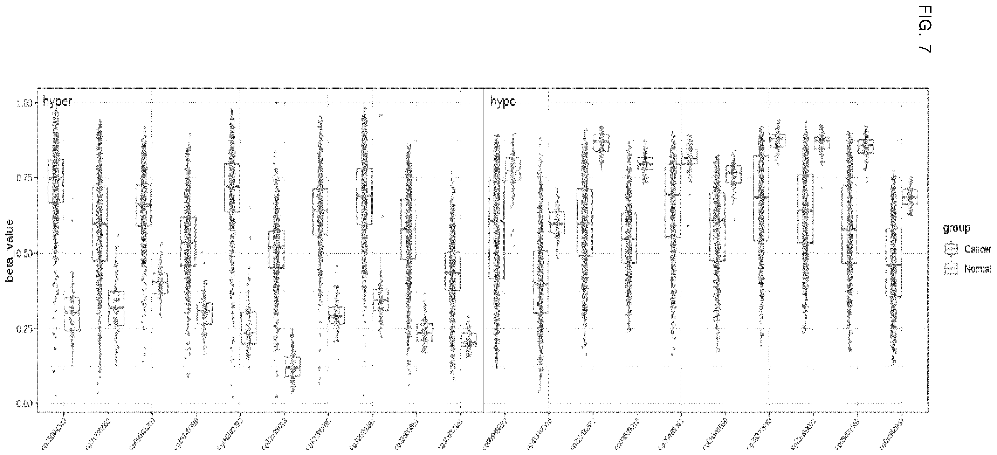

In addition, it was confirmed that the set of 20 regions and the set of 14 regions could distinguish lung cancer from surrounding tissues with high accuracy (FIG. 6 and 7).

In the table below, O in the "nCPG=14" column indicates the set of 14 regions, and X indicates a marker that was not selected from the set of 14 regions but was additionally selected from the set of 20 regions.

[Table 13]

| Chromosome | start | end | gene | illumina ProbID | nCPG=14 | gain/loss |
|---|---|---|---|---|---|---|
| chr1 | 79472407 | 79472408 | ADGRL4 | cg15084543 | O | gain |
| chr1 | 79472360 | 79472361 | ADGRL4 | cg04360793 | O | gain |
| chr1 | 156357838 | 156357839 | - | cg22353551 | O | gain |
| chr3 | 147128156 | 147128157 | ZIC4 | cg12595013 | O | gain |
| chr3 | 147130535 | 147130536 | ZIC1 | cg19029181 | O | gain |
| chr7 | 27225542 | 27225543 | HOXA11 | cg10657141 | O | gain |
| chr14 | 57265874 | 57265875 | - | cg18280830 | O | gain |
| chr1 | 157164795 | 157164796 | - | cg09504320 | X | gain |
| chr3 | 181442675 | 181442676 | SOX2-OT | cg01783662 | X | gain |
| chr5 | 92909428 | 92909429 | NR2F 1-AS1 | cg15143788 | X | gain |
| chr16 | 87958492 | 87958493 | CA5A | cg06948222 | O | loss |
| chr1 | 9896308 | 9896309 | - | cg20488341 | O | loss |
| chr2 | 10666712 | 10666713 | - | cg08846959 | O | loss |
| chr7 | 157544911 | 157544912 | PTPRN2 | cg22377978 | O | loss |
| chr8 | 978225 | 978226 | - | cg04544946 | O | loss |
| chr11 | 2033842 | 2033843 | - | cg25068071 | O | loss |
| chr19 | 57175042 | 57175043 | ZNF835 | cg08431567 | O | loss |
| chr7 | 1303350 | 1303351 | - | cg23167506 | X | loss |
| chr12 | 1943888 | 1943889 | LRTM2 | cg02505216 | X | loss |
| chr17 | 79652243 | 79652244 | ARL 16 | cg22209573 | X | loss |

**Example 9. Evaluation of Marker Panel Performance in Clinical Samples**

The performance of each of the marker sets (Tables 9 and 13) was evaluated by performing targeted EM-Seq was performed on samples from 129 lung cancer patients and 184 normal individuals shown in Table 14.

[Table 14]

| Lung Panel | **Lung cancer** | **Normal** | **Total** |
|---|---|---|---|
| **Total** | 129 | 55 | 184 |

9-1. Targeted EM-Seq

Blood was collected from the above patients, and then subjected to a first centrifugation under the conditions of 3,000 rpm, 25°C, and 10 minutes to separate only the plasma portion. Then, the plasma by separated by the first centrifugation was subjected to a second centrifugation under the conditions of 16,000g, 25°C, and 10 minutes to separate the plasma supernatant excluding the precipitate. Cell-free DNA was extracted from the separated plasma using the Mag-bind cfDNA kit, and the concentration was measured using the Qubit DS DNA HS assay kit (Thermo Fisher Scientific, USA). For the maximum amount of the extracted cfDNA, methylation conversion was performed by replacing unmethylated cytosines with uracil using ten-eleven translocation dioxygenase 2 (TET2) and APOBEC, and then a library was prepared using enzymatic methyl-seq (NEB Kit).

The concentration and size of the prepared DNA library were measured using the Qubit DS DNA HS assay kit (Thermo Fisher Scientific, USA) and Tapestation 4200 (Agilent, USA), respectively. 200 ng of the library was pooled into groups of 8 samples, hybridization was performed, and the concentration of the captured sample was measured using the high sensitivity D1000 screen tape and reagent (Agilent, USA) with Tapestation 4200 (Agilent, USA). Sequencing was performed at a final concentration of 11 pM using the Miseq Dx (Illumina) instrument in 150 paired-end mode, generating 700X depth per sample.

9-2. Evaluation of Performance

Since methylated cell-free nucleic acids were sequenced, the obtained nucleic acid fragment data are in a methylated state, and can be aligned to the human reference genome, thereby identifying methylated regions in the entire human genome. In EM-Seq data, methylated cytosine remains as cytosine, and unmethylated cytosine is converted to thymine, so that methylated regions and the degree of methylation can be identified.

First, adapter trimming and quality trimming of fastq files were performed using Trim Galore (version 0.6.6), and then nucleic acid fragment data were aligned to the human reference genome (hgl9) using the Bismark (version 0.23.0) alignment tool. Only nucleic acid fragments with a mapping quality of 10 or higher were selected using the Samtools view (version 1.11) tool, and chrl-22, X, Y were retained. Then, methylation calling was performed using bismark_methylation _extractor of Bismark (version 0.23.0).

The beta values (methylation percentage) of the tumor and normal samples were merged into a single file using the methylKit (version 1.12.0) R package, and then CpGs with an absolute value of difference exceeding 25 and a qvalue of less than 0.01 were selected using the methylKit (version 1.12.0) R package, and then the z-score was calculated using Equation 1 based on the standard deviation of the normal samples obtained in Example 7 using the beta value.

Using the calculated z-scores, the mean of z-scores, the sum of z-scores, and the SD of z-scores were calculated, and if the calculated mean of z-scores, the sum of z-scores, and the SD of z-scores exceeded the cut-off values shown in Table 8 above, the presence of lung cancer was determined. The cut-off values were the same as those in Example 7.

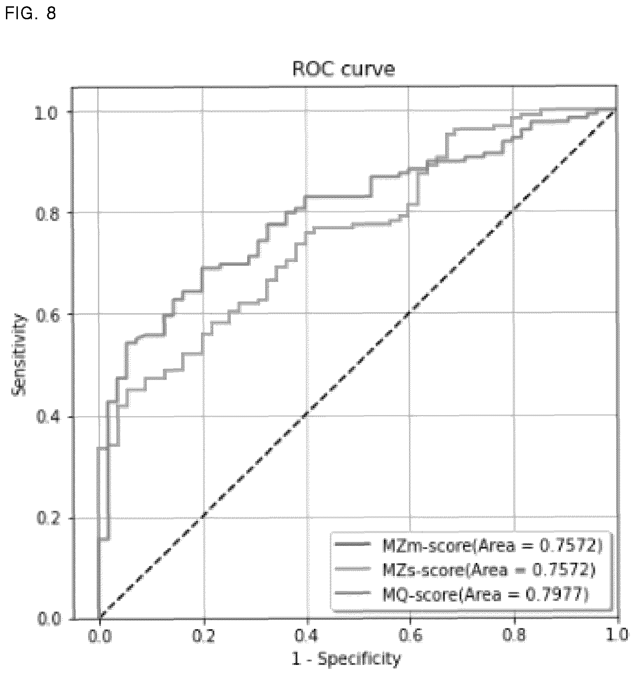

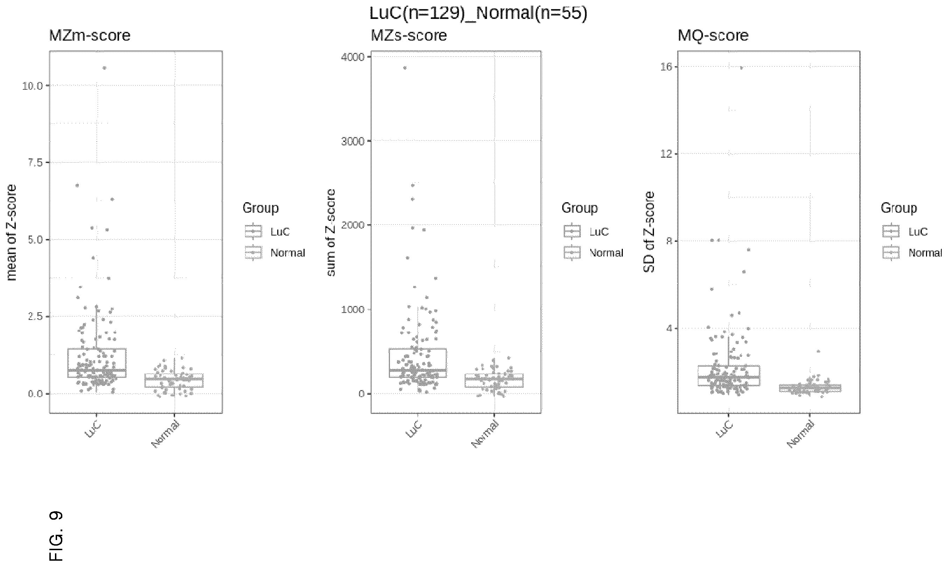

As a result, as shown in Table 15 below and FIGS. 8 and 9, it was confirmed that, when a combination of the 366 markers was used, the AUC values, which are the results of ROC analysis, were 0.75, 0.75, and 0.80 for the mean of Z-scores, the sum of Z-scores, and the SD of Z-scores, respectively.

[Table 15]

| Score | AUC |
|---|---|
| **Mean of Z-score** | 0.75 |
| **Sum of Z-score** | 0.75 |
| **SD of Z-score** | 0.80 |

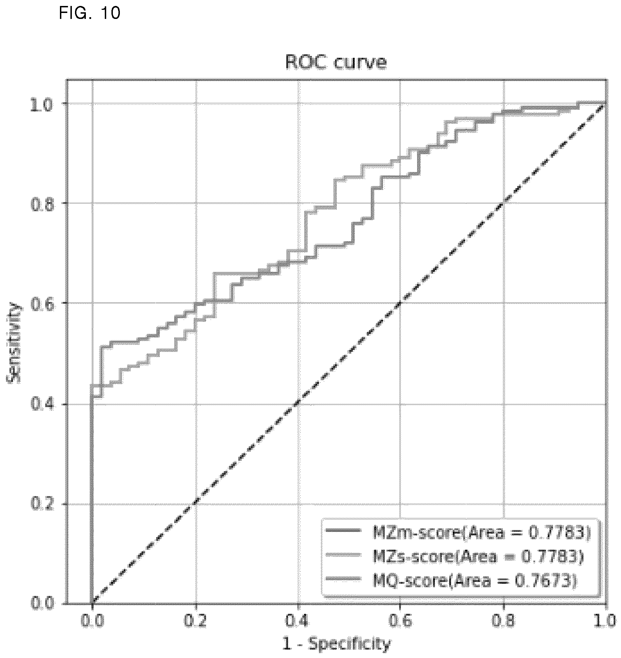

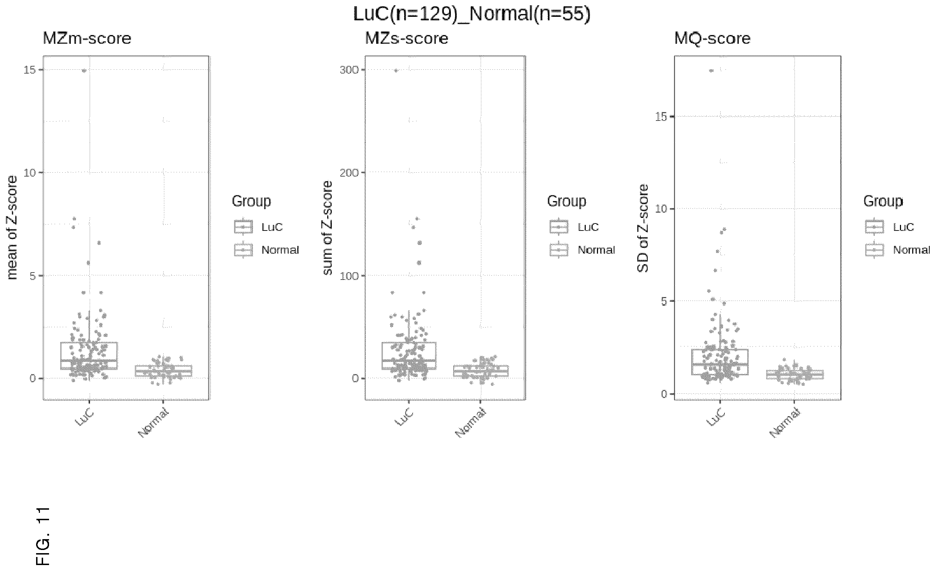

As shown in in Table 16 below and FIGS. 10 and 11, it was confirmed that, when a combination of the 20 markers was used, the AUC values, which are the results of ROC analysis, were 0.78, 0.78, and 0.77 for the mean of Z-scores, the sum of Z-scores, and the SD of Z-score, respectively.

[Table 16]

| Score | AUC |
|---|---|
| **Mean of Z-score** | 0.78 |
| **Sum of Z-score** | 0.78 |
| **SD of Z-score** | 0.77 |

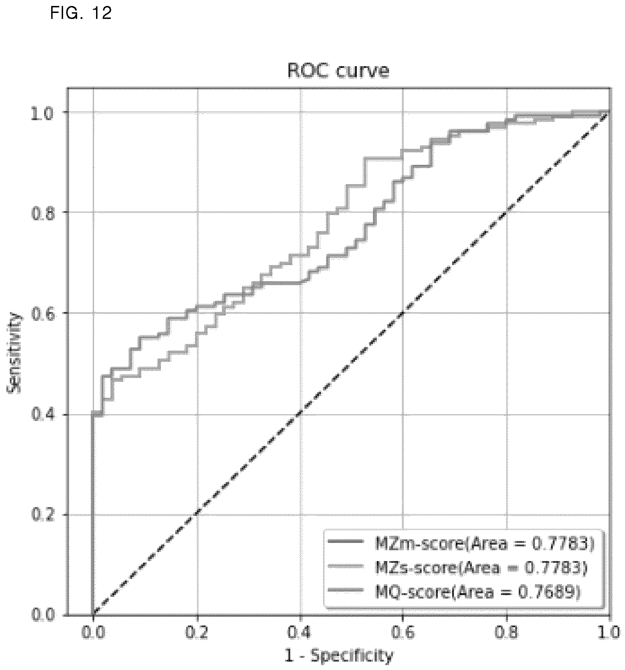

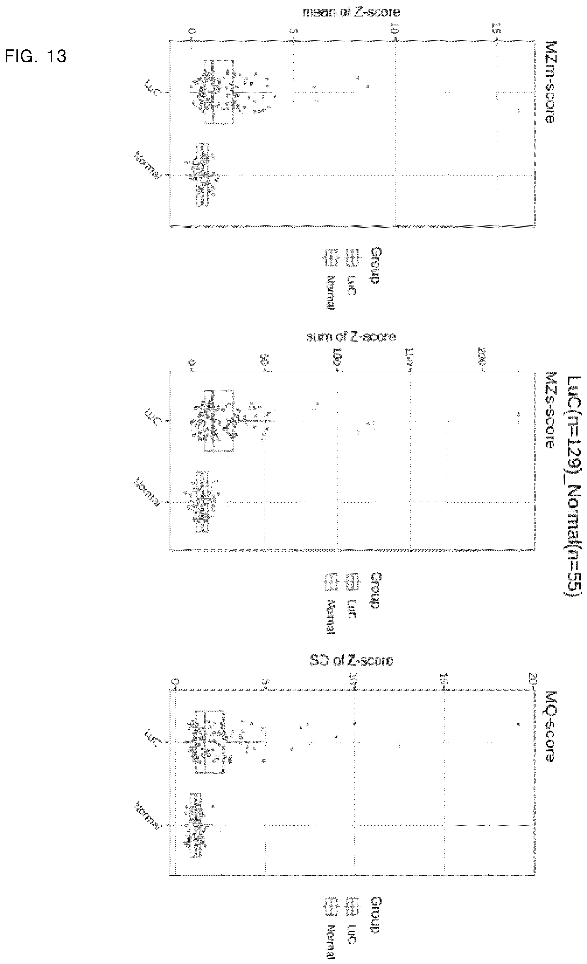

As shown in Table 17 below and FIGS. 12 and 13, it was confirmed that, when a combination of the 14 markers was used, the AUC values, which are the results of ROC analysis, were 0.78, 0.78, and 0.77 for the mean of Z-scores, the sum of Z-scores, and the SD of Z-scores, respectively.

[Table 17]

| Score | AUC |
|---|---|
| **Mean of Z-score** | 0.78 |
| **Sum of Z-score** | 0.78 |
| **SD of Z-score** | 0.77 |

Although the present invention has been described in detail with reference to specific features, it will be apparent to those skilled in the art that this description is only of a preferred embodiment thereof, and does not limit the scope of the present invention. Thus, the substantial scope of the present invention will be defined by the appended claims and equivalents thereto.

**Industrial Applicability**

The DNA methylation markers for diagnosing lung cancer according to the present invention are capable of diagnosing lung cancer with high accuracy using only DNA methylation information of a blood sample without using a lung cancer tissue sample, and thus may be useful for early diagnosis of lung cancer.

**Claims**

1. A combination of DNA methylation markers for diagnosing lung cancer, comprising DNA methylation markers shown in Table 1 below:

[Table 1]

| Chromosome | Start position | End position |
|---|---|---|
| chr1 | 79472407 | 79472408 |
| chr1 | 79472360 | 79472361 |
| chr1 | 156357838 | 156357839 |
| chr3 | 147128156 | 147128157 |
| chr3 | 147130535 | 147130536 |
| chr7 | 27225542 | 27225543 |
| chr14 | 57265874 | 57265875 |
| chr16 | 87958492 | 87958493 |
| chr1 | 9896308 | 9896309 |
| chr2 | 10666712 | 10666713 |
| chr7 | 157544911 | 157544912 |
| chr8 | 978225 | 978226 |
| chr11 | 2033842 | 2033843 |
| chr19 | 57175042 | 57175043 |

2. The combination of DNA methylation markers for diagnosing lung cancer according to claim 1, wherein the combination of DNA methylation markers for diagnosing lung cancer further comprises DNA markers shown in Table 2 below:

[Table 2]

| Chromosome | Start position | End position |
|---|---|---|
| chr1 | 157164795 | 157164796 |
| chr3 | 181442675 | 181442676 |
| chr5 | 92909428 | 92909429 |
| chr7 | 1303350 | 1303351 |
| chr12 | 1943888 | 1943889 |
| chr17 | 79652243 | 79652244 |

3. The combination of DNA methylation markers for diagnosing lung cancer according to claim 2, wherein the combination of DNA methylation markers for diagnosing lung cancer further comprises two or more DNA methylation markers selected from the group consisting of DNA markers shown in Table 3 below:

[Table 3]

| Chromosome | Start position | End position | chromosome | Start position | End position |
|---|---|---|---|---|---|
| chr1 | 38412711 | 38412712 | chr12 | 132847907 | 132847908 |
| chr1 | 63790044 | 63790045 | chr12 | 132892613 | 132892614 |
| chr1 | 75595970 | 75595971 | chr12 | 133028690 | 133028691 |
| chr1 | 79472282 | 79472283 | chr13 | 109741117 | 109741118 |
| chr1 | 79472452 | 79472453 | chr13 | 109741176 | 109741177 |
| chr1 | 110611218 | 110611219 | chr13 | 112200864 | 112200865 |
| chr1 | 119526060 | 119526061 | chr13 | 112607846 | 112607847 |
| chr1 | 119529219 | 119529220 | chr13 | 114056351 | 114056352 |
| chr1 | 119532093 | 119532094 | chr13 | 114065008 | 114065009 |
| chr1 | 119535928 | 119535929 | chr13 | 114065669 | 114065670 |
| chr1 | 119548825 | 119548826 | chr13 | 114311898 | 114311899 |
| chr1 | 170630070 | 170630071 | chr14 | 77328166 | 77328167 |
| chr1 | 214153294 | 214153295 | chr14 | 100658902 | 100658903 |
| chr1 | 214153460 | 214153461 | chr14 | 101334582 | 101334583 |
| chr1 | 214153472 | 214153473 | chr14 | 104648347 | 104648348 |
| chr10 | 81003175 | 81003176 | chr14 | 104837541 | 104837542 |
| chr10 | 103043991 | 103043992 | chr14 | 104837550 | 104837551 |
| chr10 | 118892211 | 118892212 | chr14 | 105031243 | 105031244 |
| chr11 | 31819444 | 31819445 | chr14 | 106174668 | 106174669 |
| chr12 | 54339653 | 54339654 | chr14 | 106239286 | 106239287 |
| chr12 | 85667616 | 85667617 | chr14 | 106320669 | 106320670 |
| chr12 | 85671811 | 85671812 | chr15 | 26235162 | 26235163 |
| chr12 | 85673221 | 85673222 | chr15 | 78505051 | 78505052 |
| chr13 | 79169872 | 79169873 | chr15 | 89386800 | 89386801 |
| chr13 | 112726118 | 112726119 | chr16 | 605214 | 605215 |
| chr13 | 112759719 | 112759720 | chr16 | 605347 | 605348 |
| chr14 | 29235193 | 29235194 | chr16 | 644081 | 644082 |
| chr14 | 29235196 | 29235197 | chr16 | 1052939 | 1052940 |

(continued)

| Chromosome | Start position | End position | chromosome | Start position | End position |
|---|---|---|---|---|---|
| chr14 | 29254942 | 29254943 | chr16 | 1061053 | 1061054 |
| chr14 | 57278188 | 57278189 | chr16 | 1093823 | 1093824 |
| chr14 | 99729088 | 99729089 | chr16 | 1147829 | 1147830 |
| chr14 | 101923256 | 101923257 | chr16 | 1233872 | 1233873 |
| chr16 | 66613266 | 66613267 | chr16 | 1256295 | 1256296 |
| chr16 | 86547530 | 86547531 | chr16 | 1257884 | 1257885 |
| chr19 | 11785188 | 11785189 | chr16 | 1271741 | 1271742 |
| chr19 | 12624466 | 12624467 | chr16 | 1271998 | 1271999 |
| chr19 | 22806184 | 22806185 | chr16 | 1316081 | 1316082 |
| chr19 | 58220494 | 58220495 | chr16 | 2213440 | 2213441 |
| chr19 | 58220516 | 58220517 | chr16 | 2213749 | 2213750 |
| chr19 | 58220657 | 58220658 | chr16 | 11770950 | 11770951 |
| chr19 | 58220662 | 58220663 | chr16 | 22326535 | 22326536 |
| chr19 | 58220773 | 58220774 | chr16 | 81564192 | 81564193 |
| chr2 | 45159894 | 45159895 | chr16 | 85019773 | 85019774 |
| chr2 | 63281844 | 63281845 | chr16 | 85678884 | 85678885 |
| chr2 | 66666684 | 66666685 | chr16 | 86465884 | 86465885 |
| chr2 | 73148229 | 73148230 | chr16 | 87682572 | 87682573 |
| chr2 | 74875227 | 74875228 | chr16 | 87958407 | 87958408 |
| chr2 | 119599459 | 119599460 | chr16 | 88442086 | 88442087 |
| chr2 | 119607379 | 119607380 | chr16 | 88460610 | 88460611 |
| chr2 | 119607573 | 119607574 | chr16 | 89016957 | 89016958 |
| chr2 | 171676809 | 171676810 | chr16 | 89029055 | 89029056 |
| chr2 | 172952948 | 172952949 | chr16 | 89110004 | 89110005 |
| chr2 | 176948728 | 176948729 | chr16 | 89118838 | 89118839 |
| chr2 | 176981336 | 176981337 | chr16 | 89245231 | 89245232 |
| chr2 | 176986460 | 176986461 | chr16 | 89704682 | 89704683 |
| chr2 | 176993017 | 176993018 | chr17 | 38860042 | 38860043 |
| chr2 | 177012191 | 177012192 | chr17 | 40705588 | 40705589 |
| chr2 | 177014996 | 177014997 | chr17 | 48680554 | 48680555 |
| chr2 | 177015044 | 177015045 | chr17 | 49027095 | 49027096 |
| chr2 | 177027043 | 177027044 | chr17 | 77174120 | 77174121 |
| chr2 | 177029073 | 177029074 | chr17 | 79096529 | 79096530 |
| chr2 | 177029459 | 177029460 | chr17 | 79099882 | 79099883 |
| chr2 | 177029608 | 177029609 | chr17 | 79961522 | 79961523 |
| chr2 | 182543233 | 182543234 | chr17 | 80342459 | 80342460 |
| chr20 | 43726765 | 43726766 | chr18 | 44259973 | 44259974 |
| chr3 | 138662315 | 138662316 | chr18 | 61817320 | 61817321 |
| chr3 | 147111660 | 147111661 | chr18 | 76485587 | 76485588 |

(continued)

| Chromosome | Start position | End position | chromosome | Start position | End position |
|---|---|---|---|---|---|
| chr3 | 147113918 | 147113919 | chr19 | 3936724 | 3936725 |
| chr3 | 147114406 | 147114407 | chr19 | 4552496 | 4552497 |
| chr3 | 147142182 | 147142183 | chr19 | 13394116 | 13394117 |
| chr3 | 157812226 | 157812227 | chr19 | 18085190 | 18085191 |
| chr3 | 157813327 | 157813328 | chr2 | 1133947 | 1133948 |
| chr3 | 157820591 | 157820592 | chr2 | 1880012 | 1880013 |
| chr3 | 157821262 | 157821263 | chr2 | 4931004 | 4931005 |
| chr3 | 157821994 | 157821995 | chr2 | 4931074 | 4931075 |
| chr4 | 11370466 | 11370467 | chr2 | 10638113 | 10638114 |
| chr4 | 15704680 | 15704681 | chr2 | 26800399 | 26800400 |
| chr4 | 41875470 | 41875471 | chr2 | 233198590 | 233198591 |
| chr4 | 85402870 | 85402871 | chr2 | 239544973 | 239544974 |
| chr4 | 111543401 | 111543402 | chr2 | 241083864 | 241083865 |
| chr4 | 111552040 | 111552041 | chr20 | 5451900 | 5451901 |
| chr4 | 174452835 | 174452836 | chr20 | 60120472 | 60120473 |
| chr5 | 42952113 | 42952114 | chr20 | 60456498 | 60456499 |
| chr5 | 54519023 | 54519024 | chr20 | 60456503 | 60456504 |
| chr5 | 76932016 | 76932017 | chr20 | 61625364 | 61625365 |
| chr5 | 76934327 | 76934328 | chr20 | 61695598 | 61695599 |
| chr5 | 140536238 | 140536239 | chr20 | 61755045 | 61755046 |
| chr5 | 172671754 | 172671755 | chr20 | 61792443 | 61792444 |
| chr5 | 172672766 | 172672767 | chr20 | 62079911 | 62079912 |
| chr6 | 10425648 | 10425649 | chr21 | 46420451 | 46420452 |
| chr6 | 27648004 | 27648005 | chr21 | 46677879 | 46677880 |
| chr6 | 50804147 | 50804148 | chr22 | 29704483 | 29704484 |
| chr6 | 100050791 | 100050792 | chr22 | 37769171 | 37769172 |
| chr6 | 100066698 | 100066699 | chr22 | 38506737 | 38506738 |
| chr6 | 100903817 | 100903818 | chr22 | 43835600 | 43835601 |
| chr6 | 100917397 | 100917398 | chr22 | 44708861 | 44708862 |
| chr6 | 105388731 | 105388732 | chr22 | 49137906 | 49137907 |
| chr6 | 134210946 | 134210947 | chr22 | 49376886 | 49376887 |
| chr7 | 8482235 | 8482236 | chr22 | 49487343 | 49487344 |
| chr7 | 8482325 | 8482326 | chr22 | 50470101 | 50470102 |
| chr7 | 25898688 | 25898689 | chr3 | 14615134 | 14615135 |
| chr7 | 26897202 | 26897203 | chr3 | 128182392 | 128182393 |
| chr7 | 26897253 | 26897254 | chr3 | 134146405 | 134146406 |
| chr7 | 27192056 | 27192057 | chr3 | 194078147 | 194078148 |
| chr7 | 27205217 | 27205218 | chr3 | 196065306 | 196065307 |
| chr7 | 27206073 | 27206074 | chr3 | 196065318 | 196065319 |

(continued)

| Chromosome | Start position | End position | chromosome | Start position | End position |
|---|---|---|---|---|---|
| chr7 | 27225772 | 27225773 | chr3 | 196065320 | 196065321 |
| chr7 | 39649443 | 39649444 | chr3 | 196065328 | 196065329 |
| chr7 | 96631680 | 96631681 | chr3 | 196065569 | 196065570 |
| chr7 | 96636616 | 96636617 | chr4 | 1538525 | 1538526 |
| chr7 | 96642794 | 96642795 | chr5 | 476497 | 476498 |
| chr7 | 129423053 | 129423054 | chr5 | 476646 | 476647 |
| chr7 | 153584582 | 153584583 | chr5 | 555251 | 555252 |
| chr7 | 153584597 | 153584598 | chr5 | 1003897 | 1003898 |
| chr8 | 4849522 | 4849523 | chr5 | 1119025 | 1119026 |
| chr8 | 65498812 | 65498813 | chr5 | 1140096 | 1140097 |
| chr9 | 79638117 | 79638118 | chr5 | 1202449 | 1202450 |
| chr1 | 942161 | 942162 | chr5 | 1219972 | 1219973 |
| chr1 | 1267193 | 1267194 | chr5 | 1232590 | 1232591 |
| chr1 | 1267201 | 1267202 | chr5 | 1244729 | 1244730 |
| chr1 | 2345333 | 2345334 | chr5 | 1257084 | 1257085 |
| chr1 | 2750406 | 2750407 | chr5 | 1767994 | 1767995 |
| chr1 | 2764669 | 2764670 | chr5 | 2103257 | 2103258 |
| chr1 | 2847632 | 2847633 | chr5 | 2132336 | 2132337 |
| chr1 | 2850512 | 2850513 | chr5 | 17654594 | 17654595 |
| chr1 | 2904595 | 2904596 | chr5 | 169531595 | 169531596 |
| chr1 | 2936277 | 2936278 | chr5 | 180046901 | 180046902 |
| chr1 | 2949633 | 2949634 | chr5 | 180047184 | 180047185 |
| chr1 | 3058151 | 3058152 | chr6 | 466893 | 466894 |
| chr1 | 3083061 | 3083062 | chr6 | 25218752 | 25218753 |
| chr1 | 3150935 | 3150936 | chr6 | 169740781 | 169740782 |
| chr1 | 3210325 | 3210326 | chr7 | 1403804 | 1403805 |
| chr1 | 3495611 | 3495612 | chr7 | 4841835 | 4841836 |
| chr1 | 3606892 | 3606893 | chr7 | 71177239 | 71177240 |
| chr1 | 22925489 | 22925490 | chr7 | 154542060 | 154542061 |
| chr1 | 35220814 | 35220815 | chr7 | 155191845 | 155191846 |
| chr1 | 56880606 | 56880607 | chr7 | 157085979 | 157085980 |
| chr10 | 44197967 | 44197968 | chr7 | 157502455 | 157502456 |
| chr10 | 134652276 | 134652277 | chr7 | 157776887 | 157776888 |
| chr10 | 134833372 | 134833373 | chr7 | 157968578 | 157968579 |
| chr10 | 134833874 | 134833875 | chr7 | 158030726 | 158030727 |
| chr10 | 134838656 | 134838657 | chr7 | 158331217 | 158331218 |
| chr10 | 134842688 | 134842689 | chr7 | 158799715 | 158799716 |
| chr10 | 134912012 | 134912013 | chr7 | 158820591 | 158820592 |
| chr10 | 134928457 | 134928458 | chr8 | 1140682 | 1140683 |

(continued)

| Chromosome | Start position | End position | chromosome | Start position | End position |
|---|---|---|---|---|---|
| chr10 | 134929181 | 134929182 | chr8 | 10753237 | 10753238 |
| chr10 | 135015300 | 135015301 | chr8 | 141107113 | 141107114 |
| chr10 | 135016451 | 135016452 | chr8 | 142517494 | 142517495 |
| chr10 | 135018765 | 135018766 | chr8 | 142841913 | 142841914 |
| chr10 | 135018844 | 135018845 | chr8 | 142841965 | 142841966 |
| chr11 | 393606 | 393607 | chr8 | 143201145 | 143201146 |
| chr11 | 1006121 | 1006122 | chr8 | 143208101 | 143208102 |
| chr11 | 1050848 | 1050849 | chr8 | 143262200 | 143262201 |
| chr11 | 1257516 | 1257517 | chr8 | 143333654 | 143333655 |
| chr11 | 1464300 | 1464301 | chr8 | 143509263 | 143509264 |
| chr11 | 2182540 | 2182541 | chr8 | 143535735 | 143535736 |
| chr11 | 47359115 | 47359116 | chr8 | 143605535 | 143605536 |
| chr11 | 61302186 | 61302187 | chr8 | 143625791 | 143625792 |
| chr11 | 66104485 | 66104486 | chr8 | 143915919 | 143915920 |
| chr11 | 68964113 | 68964114 | chr8 | 143915954 | 143915955 |
| chr11 | 69192623 | 69192624 | chr8 | 143961145 | 143961146 |
| chr11 | 69192697 | 69192698 | chr8 | 144203483 | 144203484 |
| chr11 | 70781118 | 70781119 | chr8 | 144366016 | 144366017 |
| chr11 | 120008998 | 120008999 | chr8 | 144416327 | 144416328 |
| chr11 | 128812875 | 128812876 | chr8 | 144801223 | 144801224 |
| chr12 | 2613914 | 2613915 | chr8 | 145697495 | 145697496 |
| chr12 | 29650795 | 29650796 | chr8 | 145758486 | 145758487 |
| chr12 | 94954828 | 94954829 | chr9 | 137718814 | 137718815 |
| chr12 | 124693173 | 124693174 | chr9 | 138018566 | 138018567 |
| chr12 | 131580615 | 131580616 | chr9 | 140041732 | 140041733 |
| chr12 | 132680972 | 132680973 | chr9 | 140333139 | 140333140 |
| chr12 | 132839436 | 132839437 | | | |

4. A method for providing information for diagnosing lung cancer, comprising steps of:

    (a) isolating DNA from a biological sample;
    (b) detecting a methylation level of the combination of DNA methylation markers according to claim 1; and
    (c) determining the presence of lung cancer if the detected DNA methylation marker level exceeds a cut-off value.

5. A method for diagnosing lung cancer, comprising steps of:

    (a) isolating DNA from a biological sample;
    (b) detecting a methylation level of the combination of DNA methylation markers according to claim 1; and
    (c) determining the presence of lung cancer if the detected DNA methylation marker level exceeds a cut-off value.

6. The method according to claim 4 or 5, wherein detecting the methylation level in step (b) is performed using any one method selected from the group consisting of PCR, methylation-specific PCR, real time methylation-specific PCR, PCR using methylated DNA-specific binding protein, quantitative PCR, PCR using methylation-specific PNA, melting curve analysis, DNA chip assay, pyrosequencing, bisulfite sequencing, and methylation next-generation sequencing.

7. The method according to claim 4 or 5, wherein the methylation level in step (b) is expressed as a beta value

8. The method according to claim 4 or 5, wherein step (c) is performed by a method comprising steps of:

(c-i) calculating a standard deviation and mean of beta values obtained from normal group samples;
(c-ii) calculating a z-score using the following equation 1;

Equation 1:

$$Z\ score = \frac{\text{methylation level in step (b)} - \text{mean of methylation levels in normal samples}}{\text{standard deviation of methylation levels in normal samples}}$$

calculating a sum, mean, or standard deviation of the calculated z-scores; and
(c-iii) determining the presence of lung cancer if the sum, mean, or standard deviation of the calculated z scores exceeds a cut-off value.

9. A composition for diagnosing lung cancer, comprising a combination of primers capable of amplifying each DNA methylation marker of the combination of DNA methylation markers according to any one of claims 1 to 3.

10. A composition for diagnosing lung cancer, comprising a combination of probes capable of specifically hybridizing to either a polynucleotide comprising at least 10 or more contiguous nucleotides, which contains a methylated base of a DNA methylation marker of the combination of DNA methylation markers according to any one of claims 1 to 3, or a polynucleotide complementary thereto.

11. A kit for diagnosing lung cancer, comprising the composition according to claim 9 or 10.

FIG. 1

FIG. 2

ROC curve

FIG. 3

LuC(n=12)_Normal(n=7)

FIG. 4

FIG. 5

FIG. 6

ROC_AUC curve

20 markers (area = 0.9983)
14 markers (area = 0.9977)

FIG. 7

FIG. 8

ROC curve

(Legend:)
- MZm-score(Area = 0.7572)
- MZs-score(Area = 0.7572)
- MQ-score(Area = 0.7977)

(Y-axis: Sensitivity)
(X-axis: 1 - Specificity)

FIG. 9

FIG. 10

ROC curve

FIG. 11

FIG. 12

ROC curve

MZm-score(Area = 0.7783)
MZs-score(Area = 0.7783)
MQ-score(Area = 0.7689)

LuC(n=129)_Normal(n=55)

FIG. 13

**EP 4 613 879 A1**

# INTERNATIONAL SEARCH REPORT

| International application No. |
|---|
| **PCT/KR2023/017173** |

| A. | CLASSIFICATION OF SUBJECT MATTER |
|---|---|
| | **C12Q 1/6886**(2018.01)i |

According to International Patent Classification (IPC) or to both national classification and IPC

| B. | FIELDS SEARCHED |
|---|---|

Minimum documentation searched (classification system followed by classification symbols)

C12Q 1/6886(2018.01); C12Q 1/68(2006.01); C12Q 1/6806(2018.01); C12Q 1/6876(2018.01); G01N 33/50(2006.01); G01N 33/574(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 폐암(lung cancer), 메틸화(methylation), 마커(marker), 진단(diagnosis), 염색체 (chromosome), 프라이머(primer), ADGRL4, ZIC4, ZIC1, PTPRN2

| C. | DOCUMENTS CONSIDERED TO BE RELEVANT |
|---|---|

| Category* | Citation of document, with indication, where appropriate, of the relevant passages | Relevant to claim No. |
|---|---|---|
| A | WO 2022-204358 A1 (FREENOME HOLDINGS, INC.) 29 September 2022 (2022-09-29)<br>See claims 12 and 23; and table 2. | 1-10 |
| A | WO 2022-023233 A1 (LES LABORATOIRES SERVIER et al.) 03 February 2022 (2022-02-03)<br>See claims 1-24. | 1-10 |
| A | KR 10-2019-0004768 A (EXACT SCIENCES DEVELOPMENT COMPANY, LLC et al.) 14 January 2019 (2019-01-14)<br>See claims 1-47. | 1-10 |
| A | JP 2017-506909 A (AGENCY FOR SCIENCE, TECHNOLOGY AND RESEARCH) 16 March 2017 (2017-03-16)<br>See entire document. | 1-10 |
| A | WO 2011-056489 A2 (ABBOTT LABORATORIES et al.) 12 May 2011 (2011-05-12)<br>See entire document. | 1-10 |

☐ Further documents are listed in the continuation of Box C.  ☑ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **05 February 2024** | **05 February 2024** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

**INTERNATIONAL SEARCH REPORT**

| International application No. |
| --- |
| **PCT/KR2023/017173** |

| Box No. II | Observations where certain claims were found unsearchable (Continuation of item 2 of first sheet) |
| --- | --- |

This international search report has not been established in respect of certain claims under Article 17(2)(a) for the following reasons:

1. ☐ Claims Nos.:
because they relate to subject matter not required to be searched by this Authority, namely:

2. ☐ Claims Nos.:
because they relate to parts of the international application that do not comply with the prescribed requirements to such an extent that no meaningful international search can be carried out, specifically:

3. ☑ Claims Nos.: **11**
because they are dependent claims and are not drafted in accordance with the second and third sentences of Rule 6.4(a).

Form PCT/ISA/210 (continuation of first sheet) (July 2022)

| INTERNATIONAL SEARCH REPORT | | International application No. |
| --- | --- | --- |
| Information on patent family members | | **PCT/KR2023/017173** |

| Patent document cited in search report | | | Publication date (day/month/year) | Patent family member(s) | | | Publication date (day/month/year) |
| --- | --- | --- | --- | --- | --- | --- | --- |
| WO | 2022-204358 | A1 | 29 September 2022 | KR | 10-2023-0162662 | A | 28 November 2023 |
| | | | | US | 2023-0178181 | A1 | 08 June 2023 |
| WO | 2022-023233 | A1 | 03 February 2022 | EP | 3945135 | A1 | 02 February 2022 |
| | | | | EP | 4189119 | A1 | 07 June 2023 |
| | | | | JP | 2023-537870 | A | 06 September 2023 |
| | | | | US | 2023-0257824 | A1 | 17 August 2023 |
| | | | | WO | 2022-023233 | A9 | 31 March 2022 |
| KR | 10-2019-0004768 | A | 14 January 2019 | CN | 109563546 | A | 02 April 2019 |
| | | | | CN | 109563546 | B | 09 September 2022 |
| | | | | CN | 116064796 | A | 05 May 2023 |
| | | | | EP | 3452616 | A1 | 13 March 2019 |
| | | | | EP | 3452616 | A4 | 22 January 2020 |
| | | | | KR | 10-2436270 | B1 | 25 August 2022 |
| | | | | US | 10385406 | B2 | 20 August 2019 |
| | | | | US | 11028447 | B2 | 08 June 2021 |
| | | | | US | 2017-0335401 | A1 | 23 November 2017 |
| | | | | US | 2019-0330702 | A1 | 31 October 2019 |
| | | | | US | 2021-0262042 | A1 | 26 August 2021 |
| | | | | WO | 2017-192221 | A1 | 09 November 2017 |
| JP | 2017-506909 | A | 16 March 2017 | CN | 106103739 | A | 09 November 2016 |
| | | | | CN | 106103739 | B | 06 December 2019 |
| | | | | EP | 3090065 | A1 | 09 November 2016 |
| | | | | EP | 3090065 | A4 | 02 August 2017 |
| | | | | EP | 3090065 | B1 | 11 December 2019 |
| | | | | JP | 6553085 | B2 | 31 July 2019 |
| | | | | US | 2016-0355886 | A1 | 08 December 2016 |
| | | | | WO | 2015-102536 | A1 | 09 July 2015 |
| WO | 2011-056489 | A2 | 12 May 2011 | CN | 102656458 | A | 05 September 2012 |
| | | | | CN | 102656458 | B | 19 October 2016 |
| | | | | CN | 104232762 | A | 24 December 2014 |
| | | | | CN | 104232762 | B | 23 November 2016 |
| | | | | CN | 106399506 | A | 15 February 2017 |
| | | | | CN | 107090490 | A | 25 August 2017 |
| | | | | EP | 2494360 | A2 | 05 September 2012 |
| | | | | EP | 2494360 | B1 | 16 August 2017 |
| | | | | JP | 2013-507987 | A | 07 March 2013 |
| | | | | JP | 2016-119900 | A | 07 July 2016 |
| | | | | JP | 2017-158573 | A | 14 September 2017 |
| | | | | JP | 6141396 | B2 | 07 June 2017 |
| | | | | KR | 10-1918004 | B1 | 13 November 2018 |
| | | | | KR | 10-2012-0093982 | A | 23 August 2012 |
| | | | | US | 10047403 | B2 | 14 August 2018 |
| | | | | US | 2011-0105341 | A1 | 05 May 2011 |
| | | | | US | 2016-0237504 | A1 | 18 August 2016 |
| | | | | US | 9291625 | B2 | 22 March 2016 |
| | | | | WO | 2011-056489 | A3 | 25 August 2011 |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 1557183

- KR 1191947

**Non-patent literature cited in the description**

- **CROSS S. et al.** *Curr. Opin. Gene Develop.*, 1995, vol. 5, 309

- **METZKER, M.** *Nature Biotechnology Reviews*, 2010, vol. 11, 31-46